(19) Europäisches Patentamt
European Patent Office
Office européen des brevets

(11) EP 3 827 966 A1

(12) EUROPEAN PATENT APPLICATION

(43) Date of publication:
02.06.2021 Bulletin 2021/22

(51) Int Cl.:
B29C 64/112 (2017.01)    B33Y 80/00 (2015.01)

(21) Application number: 20210365.1

(22) Date of filing: 27.11.2020

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
KH MA MD TN

(30) Priority: 29.11.2019 JP 2019216764

(71) Applicant: Ricoh Company, Ltd.
Tokyo 143-8555 (JP)

(72) Inventors:
• Saito, Takuya
Ohta-ku, Tokyo 143-8555 (JP)
• Matsumura, Takashi
Ohta-ku, Tokyo 143-8555 (JP)
• Niimi, Tatsuya
Ohta-ku, Tokyo 143-8555 (JP)

(74) Representative: White, Duncan Rohan
Marks & Clerk LLP
Fletcher House
Heatley Road
The Oxford Science Park
Oxford OX4 4GE (GB)

(54) **SOLID FREEFORM FABRICATION PRODUCT AND METHOD OF MANUFACTURING SOLID FREEFORM FABRICATION PRODUCT**

(57) A solid freeform fabrication product (100) includes a structure A (26) containing a soft material and having a hollow portion H (21;24) inside and a structure B (27) holding the structure A (26), wherein the layer between the structure A (26) and the hollow portion H (21;24) has a uniform thickness when the solid freeform fabrication product (100) is viewed from top.

# FIG. 3

**Description**

BACKGROUND

Technical Field

The present invention relates to a solid freeform fabrication product and a method of manufacturing a solid freeform fabrication product.

Description of the Related Art

Vascular surgery includes treatment of swollen parts (aneurysm) and shunt, cutoff, and anastomosis of blood vessels.

Catheter serving as an instrument having a wire-like form is intubated into a blood vessel in many cases of vascular surgery. Such insertion of catheter requires training for medical procedures (surgical technique). Animals or blood vessel models are used in the training if a human body is not used.

If an animal is used for medical procedure, affected areas are exposed to X-radiation to render blood vessels visible to enable catheter insertion because the blood vessels are present inside the animal body. Therefore, if training for medical procedure is repeated, the staff is excessively exposed to X radiations.

A vessel model has been proposed in JP-5140857-B1 (JP-2009-273508-A1) in which blood vessel film added by data processing is fabricated on the surface of the form data of three-dimensional blood flow by additive manufacturing or a catheter operation simulator has been proposed in JP-6385664-B1 (JP-2015-69054-A1) which is formed from transparent materials.

A hydrogel structure has been proposed in JP-2018-178069-A1 which has a hollow tubular structure made from materials having a high transmission.

The structure of JP-5140857-B1 (JP-2009-273508-A1) mentioned above has a structure A in the present disclosure so that a model having a massive dead weight cannot hold itself. As a result, it is not possible to achieve the original objective for training procedures because the model deviates from desired three-dimensional data.

The structure of JP-6385664-B 1 (JP-2015-69054-A1) mentioned above is formed of a laminate of plate-like imitated vessel flow path layers so that as the number of plates to be stacked increases if the plate-like imitated vessel flow path is a solid form, the thickness of the solid form increases. This increase in the thickness does not affect the visibility of the solid form when viewed from top. However, it significantly degrades visibility when viewed obliquely because the distance to the vessel flow path increases or the side of a plate overlaps the vessel flow path.

The structure of JP-2018-178069-A1 mentioned above incurs deformation by dead weight in the case of the structure A alone. When the structure has a block having a hollow tubular structure, the thickness between the top portion and the hollow portion is not uniform so that the model has a portion with poor visibility.

SUMMARY

According to embodiments of the present disclosure, an improved solid freeform fabrication product is provided which deforms on stress and provides good visibility to the hollow parts inside the solid freeform fabrication product when viewed from top or in an oblique direction, and is suitable as a model such as a vessel model for practicing procedures for surgical operation with a jig with which a hollow structure is pierced.

According to embodiments of the present disclosure, a solid freeform fabrication product (100) is provided which includes a structure A (26) containing a soft material and having a hollow portion H (21;24) inside and a structure B (27) holding the structure A (26), wherein the layer between the structure A (26) and the hollow portion H (21;24) has a uniform thickness when the solid freeform fabrication product (100) is viewed from top.

BRIEF DESCRIPTION OF THE SEVERAL VIEWS OF THE DRAWINGS

Various other objects, features and attendant advantages of the present invention will be more fully appreciated as the same becomes better understood from the detailed description when considered in connection with the accompanying drawings in which like reference characters designate like corresponding parts throughout and wherein:

FIG. 1 is a diagram illustrating a view of a change in the vision direction within the range of a swing when the hollow part of a structure is viewed;
FIG. 2 is a diagram illustrating a method of measuring a film thickness;
FIG. 3 is a diagram illustrating a structure free of eaves;

FIG. 4A is a diagram illustrating a structure and upward direction and FIG. 4B is a diagram illustrating the structure viewed from top;

FIG. 5A is a diagram illustrating the structure and a booleaned cuboid viewed from top and FIG. 5B is a diagram illustrating the structure and a booleaned cuboid viewed from side;

FIG. 6A is a diagram illustrating a perspective view of a set of the structure and a pedestal and FIG. 6B is a diagram illustrating the set viewed from side;

FIGS. 7A and 7B are diagrams illustrating the structure, the pedestal, and the cuboid;

FIG. 8 is a schematic diagram illustrating an example of the manufacturing process utilizing a solid freeform fabrication device for use in the method of manufacturing a hydrogel structure (solid freeform fabrication product) according to an embodiment of the present disclosure;

FIG. 9 is a schematic diagram illustrating an example in which a first liquid and a second liquid are mixed according to a liquid discharging method; and

FIG. 10 is a schematic diagram illustrating a method by which liquid A and liquid B are mixed in a form of dot in a particular mixing ratio.

The accompanying drawings are intended to depict example embodiments of the present invention and should not be interpreted to limit the scope thereof. The accompanying drawings are not to be considered as drawn to scale unless explicitly noted. Also, identical or similar reference numerals designate identical or similar components throughout the several views.

DESCRIPTION OF THE EMBODIMENTS

In describing embodiments illustrated in the drawings, specific terminology is employed for the sake of clarity. However, the disclosure of this specification is not intended to be limited to the specific terminology so selected and it is to be understood that each specific element includes all technical equivalents that have a similar function, operate in a similar manner, and achieve a similar result.

As used herein, the singular forms "a", "an", and "the" are intended to include the plural forms as well, unless the context clearly indicates otherwise.

Moreover, image forming, recording, printing, modeling, etc., in the present disclosure represent the same meaning, unless otherwise specified.

Embodiments of the present invention are described in detail below with reference to accompanying drawing(s). In describing embodiments illustrated in the drawing(s), specific terminology is employed for the sake of clarity. However, the disclosure of this patent specification is not intended to be limited to the specific terminology so selected, and it is to be understood that each specific element includes all technical equivalents that have a similar function, operate in a similar manner, and achieve a similar result.

For the sake of simplicity, the same reference number will be given to identical constituent elements such as parts and materials having the same functions and redundant descriptions thereof omitted unless otherwise stated.

Solid Freeform Fabrication Product

The solid freeform fabrication of the present disclosure has a structure A containing a soft material and having a hollow portion H inside and a structure B holding the structure A, wherein the ring structure formed between the structure A and the hollow portion H viewed from top has a uniform thickness.

The structure A and the structure B may be integrated or separable. The structure A and the structure B may be formed as an integrally molded unit. Alternatively, the structure A and the structure B can be fabricated separately and combined to obtain the solid freeform fabrication product of the present disclosure.

The solid freeform fabrication product of the present disclosure is formed of a combination unit of the structure A and the structure B. The structure A includes blood vessels and lymph channels or a part of an organ connected to them and the structure B supports the structure A as a pedestal. The structure A and the hollow portion H forms a film having a uniform thickness when viewed from the opposite side of the structure B, that is, the side on which the structure is exposed to the outside. The structure A includes a model having a part a uniform thickness added around the hollow portion H. Such a model has a uniform thickness.

The solid freeform fabrication product of the present disclosure has three features. The first feature is that the structure A deforms in response to an external force like a real organ and demonstrates flexibility owing to the soft material contained in the structure A. The second feature is that the structure A can hold the original shape regardless of dead weight because the structure B supports the structure A. If the structure A is displaced by an external force, the structure A readily returns to the original position when the external force is released. The third feature is that the visibility of the hollow portion H is the same even if the angle of view (projection angle of the focused surface from perspective) increases because the thickness of the film present between the structure A and the hollow portion H on the top part is uniform when a plane T in contact with the structure B is defined as the bottom surface.

The soft material contained in the structure A is not particularly limited as long as it has a shore A hardness of 35 or less.

Specific examples include, but are not limited to, soft resins such as polyurethane, acrylic rubber, silicone rubber and hydrogels such as polyvinyl alcohol, polyacrylamide, and polyacrylic acid. Hydrogels are preferable because its texture is similar to that of a live body. The hydrogel represents a gel including water as the main component.

The hollow portion H is not particularly limited and is preferably an inner cavity formed in a live body.

Specific examples include, but are not limited to, an artery, vena, heart, lymph channel, bile duct, cystic duct, pancreas duct, spleen duct, small intestines such as intestine duodenum, intestinum jejunum, and intestinum ileum, large intestines such as intestinal cecum, colon, intestinum rectum, and canalis analis, stomach, renal pelvis, ureter, bladder, urethral tube, womb, ovarian canal, vagina, hypophysial portal vein, pharynges, throat cavity, buccal cavity, throat, bronchus, bronchiolus, alveolar duct, acoustic duct, and nasal cavity. The hydrogel preferably contains one of artery, vena, and lymph channel in particular to insert medical equipment in a tube. The most preferable is an artery usable for training for procedures with a catheter.

Such an artery preferably has a hollow portion having a diameter of from 2.0 to 0.5 mm to practice procedures for surgical training with a catheter. The hollow portion H can be used to confirm whether a catheter having a diameter in the range mentioned above can be inserted into a tube because the hollow portion of a live body is not truly cylindrical. Alternatively, the hollow portion can be mechanically measured by an instrument such as caliper. Also, it can be measured utilizing a microscope or a one-shot 3D form measuring device (for example, device available from KEYENCE CORPORATION).

"From top" means the direction along which the model is viewed most frequently during the procedures for surgical training. In a real operation, the field of a doctor is limited in a particular direction in most cases.

In the case of a laparotomy, doctors can obtain only a perspective obtained by the incision. The perspective of doctors is limited to the vision of a camera in the case of laparoscopy or endoscope. Observation by X radiation is limited to the incidence angle of the X radiation or the detection direction. The training effect is boosted by the training for procedures because the direction obtained in a real operation can be obtained.

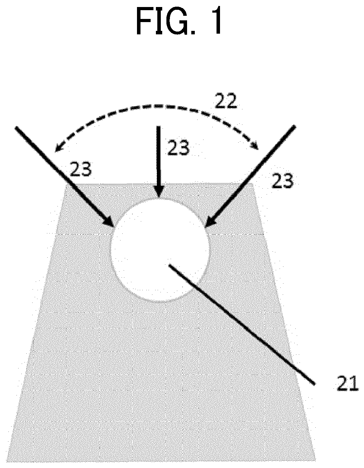

When a catheter is inserted into coronary artery, the catheter is located based on signals obtained as a result of irradiation of X radiation in a real operation. When the position of a catheter can be visibly confirmed by a training model without using X radiation, it is preferable that the catheter be located from any direction. In reality, the irradiation angle of X radiation can be changed by scanning. As illustrated in FIG. 1, the position of the catheter is visible from directions in a range of from approximately +60 degrees to approximately -60 degrees (22 illustrated in FIG. 1) by swinging instead of a single direction (23 in FIG. 1) alone. The obtained information is close to that in a real operation. If the hollow portion can be recognized in a range of from +60 degrees to -60 degrees instead of a single direction alone in the case of laparotomy and laparoscopy by swinging the view or a camera in a similar manner, the training becomes similar to a real operation.

What is required for "from top" is that the target hollow portion is visible from top and in an oblique direction in the same manner from top.

"The uniform thickness" in the present disclosure means that the value of the film thickness is within 25 to 400 percent of the average of the film thickness when the film thickness represents the thickness of the tubular structure formed by the structure A and the hollow portion H when viewed from top. This is because the film thickness changes over time in the range mentioned above owing to swelling or drying even if the film is evenly formed on the basis of the original fabrication data.

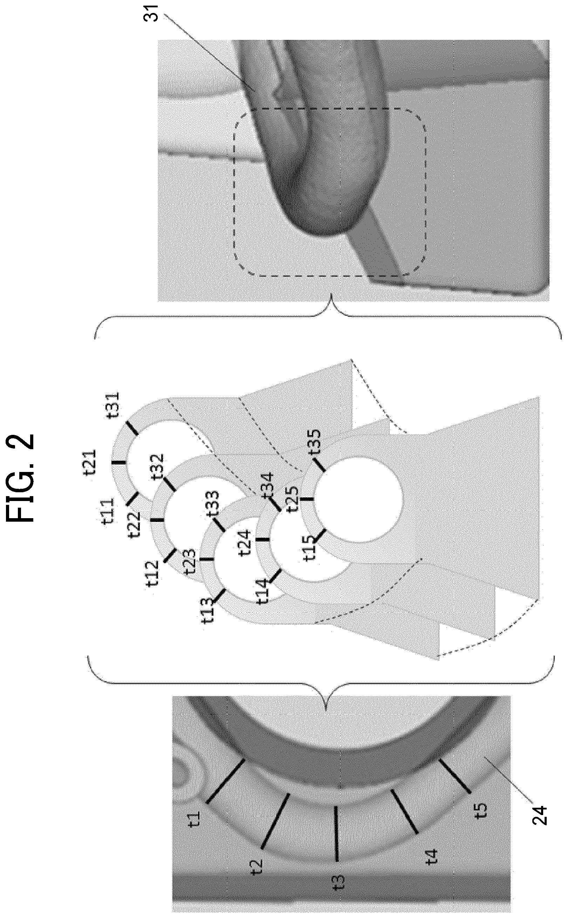

The thickness of the cross sections at t1, t2, t3, t4, and t5 perpendicular to a hollow portion 24 of a structure is measured as the film thickness as illustrated in FIG. 2.

The film thickness of the cross section crossing the structure at an angle of 60 degrees (t1n, t3n, where n represents an integer, specifically t11, t12, t13, t14, and t15, and t31, t32, t33, t34, and t35) to the perpendicular direction is also measured in addition to the perpendicular direction (t2n, where n represent an integer, specifically t21, t22, t23, t24, and t25).

The structure is cut to measure the film thickness thereof with caliper, micrometer, or microscope, with a one shot 3D form measuring device.

It is preferable that the solid freeform fabrication product furthermore include a portion of the soft material mentioned above having a moisture content of 50 percent or more, the soft material contain a mineral and a polymer, and the solid freeform fabrication product be formed of a hydrogel enclosing water in a three-dimensional network structure formed of a complex of the mineral and the polymer. The hydrogel represents a gel including water as the main component.

The structure A and the structure B holding the structure A may be separable or inseparable. When both structures can be separated from each other, the hollow portion H can be observed in all directions and the structure A can be held free of deformation when combined with the structure B. When the structure A and the structure B are combined in a physically complicated manner, the unit can be an inseparable set. The structure A is held by the structure B accordingly and free of deformation owing to its dead weight. When the structure A and the structure B are not separable, both structures can be formed inseparably integrated. If the border between the structure A and the structure B is not visible, visibility of the structures from oblique angles is enhanced.

It is preferable that the structure A and the structure B be integrally molded. If a complicated structure A and a structure B that can readily hold the structure A are separately molded to manufacture a solid freeform fabrication product, it is highly likely that either or both of the structure A and the structure B is broken when the structure A and the structure B are combined. Once combined, it is difficult to separate from each other. If the structure A and the structure B are integrated at the time when the data as the base of the form (also referred as "original fabrication data") of a fabrication product is created, the border surface is invisible in the solid freeform fabrication product of the present disclosure so that the visibility of the product tends to be good. During fabrication by a 3D printer, the original fabrication data is referred so that multiple structures combined in a complicated manner can be integrally fabricated.

The structure B is preferably free of eaves structures. Eaves structures may have an adverse impact on the fabrication product. In the cast molding, such an eaves structure caught when removed, which may damage the product. When a product with an eaves is additively manufactured with ink jetting, the eaves structure requires a support. When the support is removed, it raises a concern about increasing the roughness of the surface of the product.

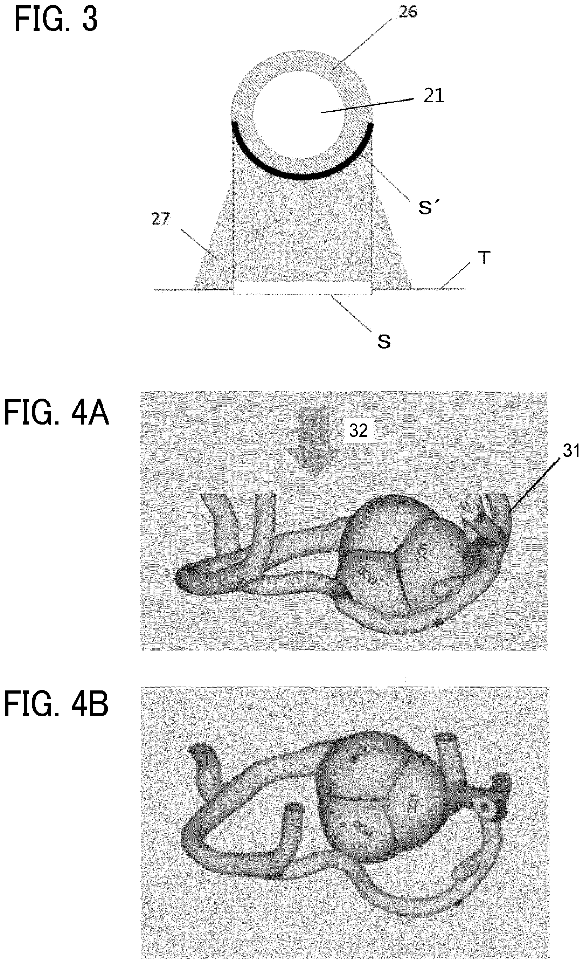

"Free of eaves" means that, as illustrated in FIG. 3, when a structure A 26 is orthogonally projected onto a plane T in contact with a structure B 27, the figure formed on the plane T is defined as a figure S and when a figure created by intersections of perpendiculars from the plane T onto the structure A 26 is defined as a figure S', the solid enclosed by the figure S and the figure S' is free of voids.

Training for Surgical Procedures Using Structure

The training for surgical procedures using the solid freeform fabrication product of the present disclosure uses either or both of catheter and endoscope.

The catheter has no particular limit and can be suitably selected to suit to a particular application. For example, catheter for angiography, balloon catheter, cerebral blood vessel catheter, cancer catheter curing, indwelling vascular catheter, indwelling suction catheter, and urethral indwelling catheter are suitable.

The endoscope has no specific limit and can be suitably selected to suit to a particular application. For example, throat cavity endoscope, bronchoscope, upper gastrointestinal endoscope, duodenoscope, enteroscope, large intestine endoscope, thoracoscope, cystoscope, cholangioscope, and angioscope are usable.

The solid freeform fabrication product of the present disclosure can be suitably applied to training for surgical procedures of catheter intubation or a simulation before surgery.

The training for surgical procedures of catheter intubation means a practice to insert a catheter into a blood vessel model and cause it to reach a target location. This training includes changing the thickness of a catheter to suit to a particular application and providing a stent, wire, and a balloon at a distal end to use it for treatment at an assumed affected part.

Selecting the optimal catheter to suit to the shape of blood vessel is a part of the training. A combinational use of the solid freeform fabrication product of the present disclosure with one or more catheters is beneficial.

It is preferable that such a training provide a texture similar to that of the inside of a real blood vessel. The solid freeform fabrication product of the present disclosure suitably deforms upon an application of an external force and returns to the original state when the external force is released. The product is expected to be highly effective because the product can show the complicated hollow portion with good visibility from different angles. In addition, it is also suitable to provide a mechanism that lets liquid flow in the hollow portion to offer a training situation in which blood is flowing.

Conventional blood vessel models are opaque in most cases. Training using such a model requires irradiation of X radiation to render the blood vessel visible. However, the training using the model of the present disclosure obviates the need for the irradiation of X radiation, thereby reducing the risk of users to be exposed to X radiation.

Lubricant for Use in Procedures for Operation

Lubricants can be optionally used to make the texture of the insertion of a catheter or endoscope into a hollow portion real as if a live body is used. It is possible to adjust the texture achieved when a catheter or endoscope is inserted into a hollow portion by applying a surfactant that can be used in a solvent, oil, and a lubricant for wet type wire drawing. These additives can be applied to the inner wall of a structure in contact with the hollow portion or let flow in a tube in a liquid form.

The solvent has no particular limit and can be suitably selected to suit to a particular application. For example, water and an organic solvent are usable. These can be used alone or in combination. Of these, organic solvents are preferable to prevent them from being absorbed in the solid freeform fabrication product of the present disclosure. Solvents having high boiling points are preferable to prevent drying when the soft material contains water.

The oil mentioned above has no specific limit and can be suitably selected to suit to a particular application. For example, synthetic oil such as mineral oil and silicone, vegetable oil, wax, and animal oil are usable.

The surfactant mentioned above has no particular limit and can be suitably selected to suit to a particular application. For example, anionic surfactants, nonionic surfactants, cationic surfactants, and amphoteric surfactants are usable. These can be used alone or in combination.

The nonionic surfactant has no particular limit and can be suitably selected to suit to a particular application.

Specific examples include, but are not limited to, polyoxyethylene alkyl ether, polyoxyethylene alkyl phenylether, polyoxyethylene alkyl phosphoric acid ester, polyoxyethylene aliphatic acid ester, sorbitan aliphatic acid ester, polyoxyethylene sorbitan aliphatic acid ester, aliphatic acid monoglyceride, sucrose aliphatic acid esters, and higher aliphatic acid alkanol amide.

The anionic surfactant has no particular limit and can be suitably selected to suit to a particular application.

Specific examples include, but are not limited to, metal salts, ammonium salts, amine salts, aminoalcohol salts, magnesium salts, and basic amino acid salts of alkyl sulfate, alkyl sulfate ether, alkyl sulfate amide ether, alkyl sulfate aryl polyether, sulfuric acid monoglyceride, alkyl sulfonate, alkylamide sulfonate, alkylaryl sulfonate, olefin sulfonate, paraffin sulfonate, alkyl sulfo succinate, alkylether sulfo succinate, alkylamide sulfo succinate, alkyl succine amide acid, alkyl sulfo acetate, alkyl phosphate, alkyl phosphate ether, acyl sarcosine, acyl isethionate, and acyl-N-acyl taurine.

The cationic surfactant has no particular limit and can be suitably selected to suit to a particular application.

Specific examples include, but are not limited to, distearyl dimethylammonium chloride, stearyldimethyl benzylammonium chloride, stearyl trimethyl ammonium chloride, behenyl trimethyl ammonium chloride, cetyl trimethyl ammonium chloride, myristyldimethyl benzyl ammonium chloride, ethyl acetate lanoline aliphatic acid amino propylethyl dimethylammonium, dicocoyl dimethylammonium chloride, lauryl trimethylammonium chloride, and ethyl sulfate branched aliphatic acid aminopropyl ethyldimethyl ammonium.

The amphoteric surfactant has no particular limit and can be suitably selected to suit to a particular application.

Examples are amide amino acid type amphoteric surfactants having an alkyl group, alkenyl group, or acyl group having 8 to 24 carbon atoms, imidazoline type amphoteric surfactant of secondary or tertiary amides, carbobetaine-based, amide betaine-based, sulfobetaine-based, hydroxy sulfobetaine-based, or amidesulfo betaine-based amphoteric surfactants having an alkyl group, alkenyl group, or acyl group having 8 to 24 carbon atoms. Specific examples include, but are not limited to, 2-alkyl-N-carboxydimethyl-N-hydroxyethylimidazolinium betaine, staryldihydroxyethylbetaine, laurylhydroxysulfobetaine, bis(stearyl-N-hydroxyethyl imidazokine)chloroacetate complex, lauryldimethylamino betaine acetate, cocoylamide propyl betaine, and cocoyl alkylbetaine.

Form of Structure B That Holds Structure A

The structure B is manufactured to hold the structure A as a live body model. One way of manufacturing the form of the structure B is as follows. 3D data editing software such as MAGICS (created by Materialise NV) is used to edit data.

Firstly, the 3D data about the structure A is obtained. The 3D data of the structure A having the hollow portion H is suitable. Alternatively, the data of the hollow portion H is obtained to add a uniform thickness by data editing.

Next, the direction of the structure A is determined. In the present disclosure, "from top (top view)" is determined as the direction in which the model is viewed most frequently during training for surgical procedures.

FIGS. 4A and 4B are diagrams illustrating the structure A 31 as a live body model including aortic valve and coronary artery of a heart and RCC represents right semilunar cusp of aortic valve, NCC represents non-coronary cusp of the aortic valve, and LCC represents a left semilunar cusp of aortic valve. FIG. 4A is a diagram illustrating a side view of the structure A 31 and FIG. 4B is a diagram thereof from top.

For the live body illustrated in FIG. 4A, the direction indicated by an arrow 32 is the most frequently viewed direction during the training for surgical procedures.

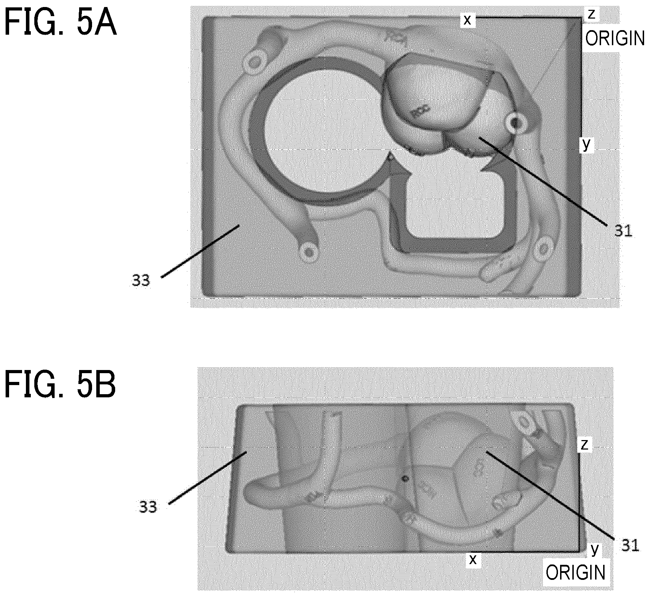

Next, a cuboid R1 33 enclosed in the structure A 31 is fabricated as illustrated in FIGS 5A and 5B. FIG. 5A is a diagram illustrating the structure A 31 and the cuboid R1 33 viewed from top and FIG. 5B is a diagram illustrating the structure A 31 and the cuboid R1 33 viewed from side. One of the surfaces of the cuboid R1 33 is necessary to be perpendicular to the top of the structure A 31. The structure A 31 is overlapped on the cuboid R1 33 and the portion having neither the structure A 31 and the cuboid R1 33 from top is deleted (booleaned). This deletion is to reduce the extra fabrication portions that prevent visions to enhance visibility of the inside.

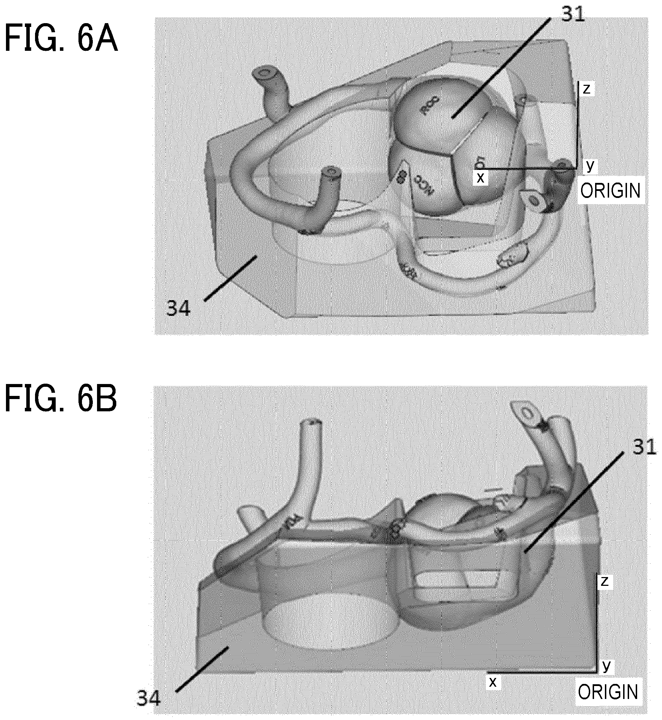

Next, the structure A 31 is overlapped on the cuboid R1 33 as illustrated in FIGS. 6A and 6B. Thereafter, the cuboid R1 33 is divided along the line passing through the structure A 31 viewed from the surface (side of the cuboid R1) parallel to the top among the surfaces of the cuboid R1 33 and the top half of the divided cuboid R1 33 is deleted to obtain a pedestal P1 34.

FIG. 6A is a diagram illustrating a perspective view of a set of the structure A 31 and the pedestal P1 34 and FIG. 6B is a diagram illustrating the set viewed from side.

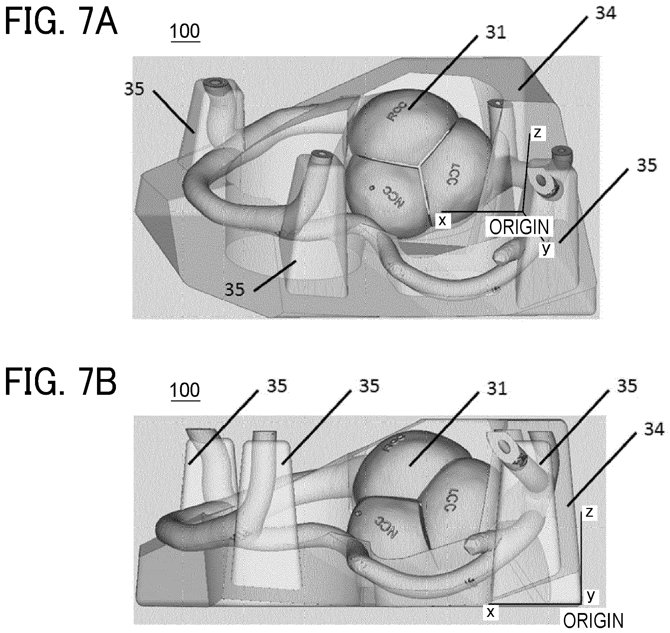

Next, as illustrated in FIGS. 7A and 7B, the structure A 31 and the pedestal P1 34 are overlapped to manufacture a cuboid R2 35 enclosing the portion floating in the air. One of the surfaces of the cuboid R2 35 is necessary to be perpendicular to the top. FIG. 7A is a diagram illustrating a perspective view of a set (solid freeform fabrication product) 100 of the structure A 31 and the pedestal P1 34 and FIG. 7B is a diagram illustrating the set 100 viewed from side.

The form of the structure B is obtained by merging the pedestal P1 34 and the cuboid R2 35 followed by boolean of the structure A 31 and the hollow portion H. When the structure A 31 and the structure B are merged, the structure A 31, the pedestal P1 34, and the cuboid R2 35 are merged followed by boolean of the hollow portion H.

The object that forms the pedestal is a cuboid but is not particularly limited as long as the surface on the top side and the surface opposite thereto are parallel to each other. Objects having a cylindrical form, truncated cone, insert form, and n polygonal column (n is 3 or an integer of 5 or greater) can be used. It is preferable that the surface on the top side from top does not protrude from the side opposite thereto when a truncated cone or insert form is used.

Data required for training for surgical procedures may be optionally edited. This data editing includes a hole connecting with the hollow portion H and forms and film thickness to adjust difficulty.

Hydrogel

The optimal material most suitable as the soft material is hydrogel.

Hydrogels are hydrophilic like live bodies and may have a moisture content of as high as from 80 to 90 percent, which is a moisture content of live bodies so that the textures, inside structure, and feeling of use can be made similar to those of organs of human bodies. Hydrogels are classified into gels derived from natural products such as gelatin, agar, and carrageen moss and synthetic gels such as polyvinyl alcohols, polyacrylicamides, and polyacrylic acids. The moisture content of the hydrogel is preferably from 50 to 98 percent by mass and more preferably from 60 to 97 percent by mass. A moisture content less than 50 percent by mass provides texture like that of a resin. In contrast, products having a moisture content greater than 98 percent by mass are too soft to hold the form on its own. Both are away from a real organ.

A moisture content of from 60 to 97 percent by mass provides a texture like that of a real organ.

The moisture content can be measured using, for example, a (heating and drying) moisture analyzer (MS-70, manufactured by A&D Company, Limited).

It is preferable that the hydrogel furthermore include a mineral and a polymer and be formed of a hydrogel enclosing water in a three-dimensional network structure formed of a complex of the mineral and the polymer. Such a hydrogel has a high degree of clearness and flexibility. Optionally, water, polymers, mineral, organic solvents, and other components are mixed by a suitable method to produce ink as hydrogel precursor. Thereafter, this ink is cured by a suitable method to prepare a hydrogel.

Polymer

There is no specific limit to the polymer and a suitable polymer is selected to suit to a particular application. For example, water-soluble polymers are preferable because the hydrogel includes water as the main component. Since the water-soluble polymer is contained, it is possible to maintain the strength of a hydrogel containing water as the main component.

Water-solubility of the water-soluble polymer means that, for example, when 1 g of the water-soluble polymer is mixed with 100 g of water and stirred at 30 degrees C, 90 percent by mass or more of the polymer is dissolved in water.

As the polymer, polymers having, for example, an amide group, an amino group, a hydroxyl group, a tetramethyl ammonium group, a silanol group, an epoxy group, etc. are suitable.

Homopolymers (monopolymer) and heteropolymers (copolymers) can be the polymer. These can be non-modified. Also, known functional groups can be introduced into these. In addition, the polymer may take a salt form. Of these, homo polymers are preferable.

The polymer can be obtained by polymerizing a polymerizable monomer. The polymerizable monomer is described in the method of manufacturing a hydrogel structure, which is described later.

The water-soluble polymer is prepared by polymerization of a polymerizable monomer. Specific examples include, but are not limited to, acrylamide, N-substituted acrylamide derivative, N,N-di-substituted acrylamide derivative, N-substituted methacrylamide derivative, and N,N-di-substituted methacrylamide derivative. These can be used alone or in combination.

When the polymerizable monomer is polymerized, water-soluble polymers having an amide group, an amino group, a hydroxyl group, a tetramethyl ammonium group, a silanol group, an epoxy group, etc. are obtained.

The water-soluble polymer having an amide group, an amino group, a hydroxyl group, a tetramethyl ammonium group, a silanol group, an epoxy group, etc. are advantageous to maintain the strength of an aqueous gel.

There is no specific limitation to the proportion of the polymer and it can be suitably selected to suit to a particular application. It is preferably from 0.5 to 20 percent by mass to the total content of the hydrogel structure.

Mineral

There is no specific limitation to the mineral and it can be suitably selected to suit to a particular application. Since the main component of the hydrogel is water, clay mineral is preferable and laminate clay minerals uniformly dispersible in water at the level of primary crystal are preferable and water swellable lamellar clay minerals are more preferable.

In the water swellable lamellar clay mineral, two-dimensional crystals having unit lattices in crystals are piled. When the water swellable lamellar clay mineral is dispersed in water, every single layer is separated to form a disk-like form crystal.

Such water swellable lamellar clay minerals have no particular limit and can be suitably selected to suit to a particular application. Examples include, but are not limited to, water swellable smectite and water swellable mica. These can be used alone or in combination. Of these, water swellable hectorite containing sodium as an interlayer ion, water swellable montmorillonite, water swellable saponite, and water swellable synthetic mica are preferable. Water swellable hectorite is more preferable because bolus having a high elasticity can be obtained. "Water swellable" means that water molecules are inserted between layers of lamellar clay mineral so that it can be dispersed in water.

The mineral can be appropriately synthesized or is commercially available.

The product commercially available are not particularly limited and can be suitably selected to suit to a particular application.

Specific examples include, but are not limited to, synthetic hectorite (LAPONITE XLG, manufactured by Rock-Wood Additives Ltd.), SWN (manufactured by Coop Chemical Ltd.), and fluorinated hectorite SWF (manufactured Coop Chemical Ltd.). These can be used alone or in combination.

The proportion of the mineral is not particularly limited and can be suitably selected to suit to a particular application. For example, it is preferably from one percent by mass to 40 percent by mass and more preferably from 1 percent by mass to 25 percent by mass to the total content of a hydrogel structure in terms of modulus of elasticity and hardness of hydrogel structure.

Water

As the water, for example, deionized water, ultrafiltered water, reverse osmosis water, pure water such as distilled water, and ultra pure water can be used.

It is suitable to dissolve or disperse other components such as organic solvents in the water to impart moisture retention, antibiotic property, or electroconductive property, and adjust hardness.

Organic Solvent

In the present disclosure, an organic solvent can be added to enhance moisture retention of the hydrogel structure.

An example of the organic solvent is a water-soluble organic solvent. The water-solubility of the water-soluble organic solvent means that the organic solvent is soluble in water in an amount of 30 percent by mass or more.

The water-soluble organic solvent is not particularly limited and can be suitably selected to suit to a particular application.

Specific examples include, but are not limited to, alkyl alcohols having one to four carbon atoms such as methyl alcohol, ethyl alcohol, n-propyl alcohol, isopropyl alcohol, n-butyl alcohol, sec-butyl alcohol, and tert-butyl alcohol, amides such as dimethylformamide and dimethylacetoamide, ketones or ketone alcohols such as acetone, methylethylketone, and diacetone alcohol, ethers such as tetrahydrofuran and dioxane, multi-valent polyols such as ethylene glycol, propylene glycol, 1,2-propane diol, 1,2-butanediol, 1,3-butanediol, 1,4-butanediol, diethylene glycol, triethylene glycol, 1,2,6-hexane triol, thioglycol, hexylene glycol, and glycerin, polyalkylene glycols such as polyethylene glycol and polypropylene glycol, lower alcohol ethers of polyols such as ethylene glycol monomethyl (or ethyl) ether, diethylene glycol methyl (or ethyl) ether, and triethylene glycol monomethyl (or ethyl) ether, alkanol amines such as monoethanol amine, diethanol amine, and triethanol amine, N-methyl-2-pyrrolidone, 2-pyrrolidone, and 1,3-dimethyl-2-imidazoline. These can be used alone or in combination. Of these, in terms of moisture retention, polyols, glycerin, and propylene glycol are preferable and glycerin and propylene glycol are more preferable.

The proportion of the organic solvent is preferably from 10 to 50 percent by mass to the total content of the hydrogel structure. When the proportion is not less than 10 percent by mass, the hydrogen is sufficiently prevented from being dried. In addition, when the proportion is not greater than 50 percent by mass, mineral is uniformly dispersed.

Other Components

The other optional ingredients have no particular limit and can be suitably selected to suit to a particular application. For example, stabilizers, surface treatment chemicals, polymerization initiators, colorants, viscosity modifiers, cohesion imparting agents, antioxidants, anti-aging agents, cross-linking promoters, ultraviolet absorbents, plasticizers, preservatives, dispersants, and surfactants.

Method of Manufacturing Solid freeform fabrication product

The method of manufacturing a solid freeform fabrication product of the present disclosure is not particularly limited as long as a solid freeform fabrication product including the structure A and the structure B can be obtained. One way of manufacturing the solid freeform fabrication product is molding a core imitating the hollow portion H, placing the core, infusing a precursor into the core, and taking out of the core after curing to obtain a structure like the shell mold molding. Alternatively, such a structure can be directly fabricated using a 3D printer. Direct fabrication using a 3D printer is preferable to manufacture a structure having high quality with complicate and fine hollow portion H because this fabrication process obviates the need for removing the core.

Moreover, the solid freeform fabrication product mentioned above is preferably manufactured by additive manufacturing (material jetting) in which a precursor (first liquid precursor) to obtain a soft material is discharged from an inkjet head followed by exposing the precursor to ultraviolet radiation. When a support is formed in the hollow portion H, the same material as the material for a model is used to form the support in stereolithography (SLA) and digital light processing (DLP) so that removing the support is difficult. Therefore, the multi-material jetting method is preferable because it can change the material for a support from that for a model and remove the support in the post-processing. One way of the post-processing is plasticization/liquefaction with heat or a solvent. A preferable post-processing is to liquidize a support with heat. This processing optionally includes cleaning and other processes.

The solid freeform fabrication product is preferably manufactured by additive manufacturing in which a precursor (second liquid precursor) of a support forming material is discharged from an inkjet head followed by exposing the precursor to ultraviolet radiation. If the method of fabricating a model is different from the method of fabricating a support, the time for fabrication significantly increases. It is preferable to utilize ultraviolet radiation to cure a support forming material, which is the same manner as for the model material, to make the fabrication time per layer short.

Below is a detailed description of the method of manufacturing a structure, in particular a hydrogel structure, according to the material jetting method mentioned above.

Additive Manufacturing Process and Layer Forming Device

The additive manufacturing process includes discharging a hydrogel forming material containing water and a polymerizable monomer and removing a support forming material to be removed later to form a layer formed of these materials.

The support forming material is applied to a site different from that of the hydrogel forming material and forms a support to support the hydrogel structure portion after it cures. To form a hollow structure in the present disclosure, the upper part of the hollow structure is supported by the corresponding support during additive manufacturing. "Site different from that of the hydrogel liquid precursor" means that the application position of the support forming material does not overlap the application position of the hydrogel forming material. Both materials may be adjacent to each other.

The method of applying the forming material as the additive manufacturing process has no particular limit as long as liquid droplets are applied to a target site with a suitable precision and it can be suitably selected to suit to a particular application. For example, a dispenser method, a spray method, and an inkjet printing method can be suitably selected to suit to a particular application. Known devices are suitably used to execute these methods.

Of these, the dispenser method has an excellent quantitative property but difficulty in achieving a wide application area. The spray method is capable of simply forming a fine discharging material, has a wide application area, and demonstrates excellent applicability but the quantitative property thereof is poor, which causes scattering due to the spray stream. For this reason, in the present disclosure, the inkjet printing method is particularly preferable. The inkjet printing method has a good quantitative property in comparison with the spray method and a wider application area in comparison with the dispenser method. Accordingly, the inkjet printing method is preferable to accurately and efficiently form a complex solid shape.

When utilizing the inkjet printing method, there are provided nozzles capable of discharging the forming materials. As the nozzle, nozzles for use in a known inkjet printer can be suitably used.

Hydrogel Forming Material (Hydrogel Precursor)

The hydrogel forming material contains water and a polymerizable monomer. It also preferably contains a mineral and an organic solvent and furthermore optionally includes a polymerizable monomer and other optional components.

As water, the mineral, the organic solvent, and the other optional components, the same as those for the hydrogel structure mentioned above can be used.

Polymerizable Monomer

The polymerizable monomer includes a compound having at least one unsaturated carbon and carbon bond. A polymerizable monomer polymerized upon application of active energy rays such as ultraviolet radiation and electron beams is preferable.

For example, a mono-functional monomer and a polyfunctional monomer are suitable as the polymerizable monomer. These can be used alone or in combination.

The polyfunctional monomer includes, for example, a bi-functional monomer, a tri-functional monomer, and a tetra- or higher functional monomer.

The mono-functional monomer is a compound having a single unsaturated carbon-carbon bond. Examples are acrylamides, N-substituted acrylamide derivatives, N,N-disubstituted acrylamide derivatives, N-substituted methacrylamide derivatives, N,N-disubstituted methacrylamide derivatives, and other mono-functional monomers. These can be used alone or in combination.

The N-substituted acrylamide derivatives, N,N-di-substituted acrylamide derivatives, N-substituted methacrylamide derivatives, and N,N-di-substituted methacrylamide derivatives include, for example, N,N-dimethyl acryl amide (DMAA) and N-isopropyl acryl amide.

Specific examples of the mono-functional polymerizable monomer include, but are not limited to, 2-etylhexyl(meth)acrylate, 2-hydroxyethyl(meth)acrylate, 2-hydroxypropyl(meth)acrylate, acryloylmorphorline (ACMO), caprolactone-modified tetrahydrofurfuryl(meta)acrylate, isobonyl(meth)acrylate, 3-methoxybutyl(meth)acrylate, tetrahydro furfuryl(meth)acrylate, lauryl(meth)acrylate, 2-phenoxyethyl (meth)acrylate, isodecyl(meth)acrylate, isooctyl(meth)acrylate, tridecyl(meth)acrylate, caprolactone(meth)acrylate, ethoxyfied nonylphenol(meth)acrylate, and urethane(meth)acrylate. These can be used alone or in combination.

Water-soluble polymers having an amide group, an amino group, a hydroxyl group, a tetramethyl ammonium group, a silanol group, an epoxy group, etc. can be obtained by polymerizing the mono-functional monomers mentioned above.

Water-soluble polymers having an amide group, an amino group, a hydroxyl group, a tetramethyl ammonium group, a silanol group, an epoxy group, etc. are advantageous to maintain the strength of a blood vessel model.

Specific examples of the bi-functional monomer include, but are not limited to, tripropylene glycol di(meth)acrylate, triethylene glycol di(meth)acrylate, tetraethylene glycol di(meth)acrylate, polypropylene glycol di(meth)acrylate, neopentyl glycol hydroxy pivalic acid ester di(meth)acrylate, hydroxypivalic acid neopentyl glycol ester di(meth)acrylate, 1,3-butanediol di(meth)acrylate, 1,4-butanediol di(meth)acrylate, 1,6-hexanediol di(meth)acrylate, 1,9-nonane diol(meth)acrylate, diethylene glycol di(meth)acrylate, neopentyl glycol di(meth)acrylate, tripropylene glycol di(meth)acrylate, caprolactone-modified hydroxy pivalic acid neopentyl glycol ester di(meth)acrylate, propoxinated neopentyl glycol di(meth)acrylate, ethoxy-modified bisphenol A di(meth)acrylate, polyethylene glycol 200 di(meth)acrylate, polyethylene glycol 400 di(meth)acrylate, and methylenebis acrylamide. These can be used alone or in combination.

Specific examples of the tri-functional monomers include, but are not limited to, trimethylol propane tri(meth)acrylate, pentaerythritol tri(meth)acrylate, tirallyl isocyanate, tris(2-hydroxyethyl)isocyanulate tri(meth)acrylate, ethoxyfied trimethylol propane tri(meth)acrylate, propoxyfied trimethylol propane tri(meth)acrylate, and propoxyfied glyceryl tri(meth)acrylate. These can be used alone or in combination.

Specific examples of the tetra- or higher monomers include, but are not limited to, pentaerythritol tetra(meth)acrylate, ditrimethylol propanetetra(meth)acrylate, dipentaerythritol hydroxypenta(meth)acrylate, ethoxyfied pentaerythritol tetra (meth)acrylate, penta(meth)acrylate ester, and dipentaerythritol hexa(meth)acrylate. These can be used alone or in combination.

The proportion of the mono-functional monomer is not particularly limited and can be suitably selected to suit to a particular application. For example, it is preferably from 1 to 10 percent by mass and more preferably from 1 to 5 percent by mass to the total content of the hydrogel forming material. When the proportion is in the range of from 1 to 10 percent by mass, dispersion stability of the mineral in the hydrogel forming material is maintained and drawing property of a hydrogel structure is enhanced. The drawing property means that when a hydrogel structure is drawn, the hydrogel structure is not fractured (broken) but extended.

The proportion of the poly-functional monomer is preferably from 0.001 to 1 percent by mass and more preferably from 0.01 to 0.5 percent by mass to the total content of the hydrogel forming material. When the proportion is in the range of from 0.001 to 1 percent by mass, it is possible to control the modulus of elasticity and hardness of the obtained hydrogel structure in a suitable range.

The proportion of the polymerizable monomer is preferably from 0.5 to 20 percent by mass to the total content of the hydrogel forming material. When the proportion is from 0.5 to 20 percent by mass, the strength of the hydrogel structure can be closer to that of a human internal organ.

Polymerization Initiator

There is no specific limitation to the polymerization initiator and it can be suitably selected to suit to a particular application. Example are polymerization initiators and thermal polymerization initiators.

As the photopolymerization initiator, any material can be used which produces a radical upon irradiation of light (ultraviolet rays in a wavelength range of 220 to 400 nm).

The photopolymerization initiator has no particular limit and can be suitably selected to suit to a particular application.

Specific examples include, but are not limited to, acetophenone, 2,2-diethoxyacetophenone, p-dimethylaminoacetone, benzophenone, 2-chlorobenzophenone, p,p'-dichlorobenzophenone, p,p-bisdiethylamonobenzophenoen, Michler's Ketone, benzyl, benzoin, benzoin methylether, benzoin ethylether, benzoin isopropylether, benzoin-n-propylether, benzoin isobutyl ether, benzoin-n-butyl ether, benzylmethyl ketal, thioxanthone, 2-chlorothioxanthone, 2-hydroxy- 2-methyl-1-phenyl-1-one, 1-(4-isopropylphenyl)-2-hydroxy-2-methylpropane-1-one, methylbenzoyl formate, 1-hydroxy cyclohexyl phenylketone, azobisisobutylo nitrile, benzoylperoxide, and di-tert-butylperoxide. These can be used alone or in combination.

The thermal polymerization initiator has no particular limitation and can be suitably selected to suit to a particular application. Examples thereof are azo-based initiators, peroxide initiators, persulfate initiators, and redox (oxidation- reduction) initiators. These can be used alone or in combination. Of these, peroxide initiators are preferable.

There is no specific limitation to the peroxide initiator and it can be suitably selected to suit to a particular application.

Specific examples include, but are not limited to, potassium persulfate, sodium persulfate, ammonium persulfate, peroxo sodium pyrosulfate, and peroxo potassium pyrosulfate. These can be used alone or in combination. Of these, potassium peroxo pyrosulfate is preferable.

Curing process and Curing Device

The curing process includes exposing a predetermined region of the hydrogel forming material layer and the support forming material layer formed with the curing device to active energy rays to cure the region.

As the curing device to cure the layers, for example, an ultraviolet (UV) lamps, electron beams are used. The curing device preferably includes a mechanism to remove ozone.

The UV lamp includes, for example, a high pressure mercury lamp, an ultra high pressure mercury lamp, a metal halide lamp, and an ultraviolet light-emitting diode (UV-LED).

The ultra-high pressure mercury lamp is a point light source but if the DeepUV type combined with an optical system to have a high light use efficiency rate is used, the lamp is capable of emitting light in a short-wavelength range.

Since the metal halide lamp has a wide range of wavelength, it is suitable for colored materials. Halogenated materials of metal such as Pb, Sn, and Fe are used therefor and can be selected to suit to absorption spectrum of a polymerization initiator. The lamp for use in the curing has no particular limit and can be suitably selected to suit to a particular application. Lamps commercially available can be used. Examples are H lamp, D lamp, and V lamp (manufactured by Fusion System).

The emission wavelength of UV-LED is not particularly limited and can be suitably selected to suit to a particular application.

In general, wavelengths of 365 nm, 375 nm, 385 nm, 395, nm and 405 nm are used. Taking into account the impact on the color of a solid freeform fabrication product, short wavelength emission is advantageous to increase absorption of a polymerization initiator. When a hydrogel readily affected by heat energy is used as the material for the solid freeform fabrication product of the present disclosure, it is preferable to use UV-LED as the UV lamp because it produces less heat.

The cured hydrogel material layer is preferably a hydrogel which contains water and ingredients soluble in the water in a three-dimensional network structure formed by complexing a polymer and a mineral. The hydrogel has good expansibility and can be peeled off without breakage at once, so that the treatment after fabrication is significantly simplified.

Material for Support Forming Material

The support is fabricated together with a solid freeform fabrication product to prevent degradation of fabrication property caused by dripping and flexure and removed in the end.

The support has no particular limit as long as it can support the hydrogel structure of the present disclosure. It is preferable to use a material having a solubility in a solvent or a material liquefied as a result of phase change by heating to remove the support present in the hollow portion after lamination. Since the hydrogel structure of the present disclosure is a hydrogel, it is preferable to avoid immersion in water to remove the support because the fabricated product may swell. For this reason, it is preferable to select a support forming material soluble in a solvent in which the hydrogel is not dissolved. In addition, the support forming material is preferably solid at 25 degrees C and is phase-changed into liquid at 50 degrees C. When the support forming material is a phase-changeable material, the support is readily removed after the hydrogel structure is formed.

In addition, the support forming material (core part forming material) used to support the inside of the hollow portion in the hydrogel structure of the present disclosure and the support forming material used to support the exterior of the structure can be the same or different from each other. Also, it is not necessary to fill the inside of the hollow portion with a support. Any minimal support is allowed which can support the hollow portion. Such a minimal support is efficient in comparison with a support which fills the entire of the inside of the hollow portion.

The support forming material contains a polymerizable monomer and other optional material such as a polymerization initiator and a colorant. The same materials mentioned above for the hydrogel forming material can be used as for the support forming materials.

The phase-changeable material includes, but is not limited to, liquid before it cures and solidified upon irradiation of active energy rays such as ultraviolet radiation like the case of the hydrogel. The materials are suitable which are solid at room temperature (25 degrees C) and liquid at 60 degrees C.

In one embodiment, it is preferable to contain a mono-functional ethylenic unsaturated monomer (A) (hereinafter referred to as monomer (A)) having a straight chain having 14 or more carbon atoms, a polymerization initiator (B), and a solvent (C) and more preferable to furthermore contain a solvent (D) in which the monomer (A) is poorly dissolved.

Mono-functional Ethylenic Unsaturated Monomer (A) Having Straight Chain Having 14 or More Carbon Atoms

The mono-functional ethylenic unsaturated monomer (A) having a straight chain having 14 or more carbon atoms has no particular limit and can be suitably selected to suit to a particular application.

Specific examples include, but are not limited to, acrylate such as stearylacrylate and docosylacrylate; methacrylate such as stearylmethacrylate and docosylmethacrylate, acylamide such as palmityl acrylamide and starylacrylamide, and vinyl such as vinylstearate and vinyl docosylate. These can be used alone or in combination.

Of these, acrylates and acrylamide derivatives are preferable in terms of optical reactiveness and stearyl acrylates are more preferable to enhance solubility in a solvent.

Examples of the polymerization reaction of the monomer (A) are radical polymerization, ion polymerization, coordination polymerization, and ring-opening polymerization. Of these, in order to control polymerization reaction, radical polymerization is preferable. For this reason, the monomer (A) having a hydrogen bond power is preferably ethylenic unsaturated monomers. Of these, in terms of solubility, mono-functional ethylenic unsaturated monomers are preferable.

Polymerization Initiator (B)

The polymerization initiator (B) has no specific limit and can be suitably selected to suit to a particular application. It includes thermal polymerization initiators and photopolymerization initiator. Of these, photopolymerization initiators are preferable to fabricate a solid freeform fabrication product.

As the photopolymerization initiator, it is possible to use any material which produces a radical at irradiation of light (in particular, ultraviolet radiation in a wavelength range of 220 to 400 nm).

Specific examples of the photopolymerization initiator include, but are not limited to, acetophenone, 2,2-diethoxyacetophenone, p-dimethylaminoacetophenone, benzophenone, 2-chlorobenzophenone, p,p'-dichlorobenzophenone, p,p-bisdiethylamonobenzophenoen, Michler's Ketone, benzyl, benzoin, benzoin methylether, benzoin ethylether, benzoin isopropylether, benzoin-n-propylether, benzoin isobutyl ether, benzoin-n-butyl ether, benzylmethyl ketal, thioxanthone, 2-chlorothioxanthone, 2-hydroxy-2-methyl-1-phenyl-1-one, 1-(4-isopropylphenyl)-2-hydroxy-2-methylpropane-1-one, methylbenzoyl formate, 1-hydroxy cyclohexyl phenylketone, azobisisobutylo nitrile, benzoylperoxide, and di-tert-butylperoxide. These can be used alone or in combination. It is preferable to select a photopolymerization initiator depending on the ultraviolet radiation wavelength of an ultraviolet radiation irradiator.

Solvent (C) Capable of Dissolving Monomer (A)

The solvent (C) has no particular limit as long as the solvent (C) can dissolve the monomer (A) and can be suitably selected to suit to a particular application. In order to prevent a significant decrease of crystallinity of a polymer side chain, it is preferable to have a straight chain having six or more carbon atoms.

Specific examples of the solvent (C) having a straight chain having six or more carbon atoms include, but are not limited to, esters such as hexyl acetate and octyl acetate and alcohols such as hexanol, decanol, and dodecanol. Of these, alcohol having a straight chain is preferable in order to enhance the support power to the modeling material of a cured object. It is possible to structure a hydrogen bond by a hydroxyl group while maintaining crystallinity of the polymer side chain. Moreover, alcohol having a straight chain having at least one hydroxyl group bonded to the primary carbon is preferable and 1-dodecanol is more preferable because it can prevent inhibition of crystallinity.

Solvent (D) in Which Monomer (A) Is Not Readily Dissolved

The solvent (D) is added in order to relieve the warpage of a support to be fabricated. If a solvent little or never soluble in a monomer is added, the internal stress occurring during curing is considered to be distributed.

The solvent (D) has no particular limit as long as it can little or never dissolve the monomer (A) and can be suitably selected to suit to a particular application. When the monomer (A), the solvent (C), and the solvent (D) are mixed, the solvent (D) is preferably present as liquid compatible in a 60 degree C environment. Moreover, polyol that can remain in a cured object without inhibiting crystallinity of the polymer side chain and decrease viscosity as ink for supporting material is more preferable.

Specific examples of the polyol include, but are not limited to, polyethers such as polyethylene glycol (PEG), polypropylene glycol (PPG), polybutylene glycol, a copolymer of ethylene oxide and propylene oxide, a copolymer of ethylene oxide and butylene oxide, and polytetramethylene ether glycol (PTMEG), polyesters such as polycarprolactone diol (PCL), polycarbonate diol, and polyester polyol formed of polyol and polybasic acid, castor oil, and acrylic polyol. These can be used alone or in combination. Of these, polypropylene glycol is preferable.

As the copolymer, a block copolymer, a random copolymer, or a combination thereof can be used in combination.

The degree of polymerization of polyol has no particular limit and can be suitably selected to suit to a particular application. For example, the degree of polymerization is preferably from 10 to 10,000, more preferably from 100 to 5,000, and particularly preferably from 1,000 to 3,000. When the degree of polymerization is 10 or greater, the polyol is not vaporized at heating and can remain present in a cured object. In addition, when the degree of polymerization is 10,000 or less, the polyol can be present in liquid without excessively increasing viscosity at 60 degrees C.

The criteria of capability of dissolving the monomer (A) is determined based on whether the monomer (A) having a proportion of 1 percent by mass of the solvent can be dissolved therein. That is, the solvent (C) can dissolve the monomer (A) having 1 percent by mass or more of the dead weight of the solvent (C) while the solvent (D) cannot dissolve the monomer (A) having 1 percent by mass or more of the dead weight of the solvent (D).

The determination can be made whether or not non-dissolved monomer (A) remains after the monomer (A) at 1 percent by mass is loaded in the solvent (C) or the solvent (D) followed by stirring for 12 hours.

The support forming material (active energy ray curable liquid composition) preferably contains the monofunctional ethylenic unsaturated monomer (A) having a straight chain having 14 or more carbon atoms at a proportion of from 20 to 70 percent by mass and more preferably from 30 to 60 percent by mass.

The active energy ray curable liquid composition of the present disclosure preferably contains the polymerization initiator (B) in an amount of from 0.5 to 10 percent by mass and more preferably from 3 to 6 percent by mass.

The active energy ray curable liquid composition of the present disclosure preferably contains the solvent (C) that can dissolve the monomer (A) in an amount of from 20 to 70 percent by mass and more preferably from 30 to 60 percent by mass.

The active energy ray curable liquid composition of the present disclosure preferably contains the solvent (D) that can poorly dissolve the monomer (A) at a proportion of from 0 to 40 percent by mass and more preferably from 10 to 30 percent by mass. When the proportion is from 0 to 40 percent by mass, warpage of the support can be relieved while the support maintains its form. If the proportion is outside the range specified above, the support tends to be deformed due to the dead weight of the hydrogel structure and the external force applied during fabrication.

In addition, when the following relation is satisfied, warpage can be reduced while securing sufficient compression stress:

$$60 < \{(Wc + Wd)/(Wa + Wc + Wd)\} < 75$$

In the relation, Wa represents the mass of the monomer (A), Wc represents the mass of the solvent (C), and Wd represents the mass of the solvent (D).

To obtain a cured object from the support forming liquid material, for example, it is preferable to expose the support forming liquid material to ultraviolet radiation at an amount of 200 mJ/cm$^2$ or greater using an ultraviolet irradiator. The same device as the device for use in curing the hydrogel structure can be used as the ultraviolet irradiator.

It is preferable that the temperature and humidity of a fabrication space be controlled from the beginning to the end of fabrication. This control is to prevent moisture absorption or drying of a fabricated object or solidification of a precursor.

Specifically, the temperature is 25 degrees C or lower and the moisture is within -5 to +5 percent of a target RH value. It is more preferable when the target RH value is 95 percent.

In addition, it is necessary to shield the surrounding in order not to leak ultraviolet radiation emitted from an ultraviolet irradiator during fabrication. The shielding structure may shield all the light or selectively shield ultraviolet radiation.

When the monomer (A) is irradiated with ultraviolet radiation together with the polymerization initiator (B), the monomer (A) becomes a polymer and the solvent (C) is maintained in the polymer. The polymer (A) is solidified when the carbon chain is arranged in a 25 degree C environment. If the solvent (C) is maintained in the polymer (A), contraction and warpage attributable to crystallization can be reduced. In addition, the solvent (C) preferably has a straight chain having six or more carbon atoms in terms of curability.

In addition, the solvent (C) capable of dissolving the monomer (A) is preferably a non-reactive compound non-reactive to the polymerization initiator (B).

In the present disclosure, the solvent (C) capable of dissolving the monomer (A) means a solvent in which the monomer (A) is dissolved to form a uniform liquid.

In the present disclosure, the non-reactive compound is not chemically reactive even if it is exposed to ultraviolet radiation.

If the solvent (C) is non-reactive, it does not react under the presence of a photopolymerization initiator so that polymerization reaction of monomers and crystallization of the polymer side chain are not inhibited. Therefore, the non-reactive solvent (C) is preferable.

Surface Tension

The surface tension of the support forming material in the present disclosure has no particular limit and can be suitably selected to suit to a particular application. For example, the surface tension is preferably from 20 to 45 mN/m and more preferably from 25 to 34 mN/m at 25 degrees C. When the surface tension is 20 mN/m or greater, it is possible to prevent unstable jetting (such as deviation of jetting direction and no jetting) during fabrication. When the surface tension is 45 mN/m or less, a jetting nozzle for fabrication can be completely filled with liquid. The surface tension can be measured by equipment such as a surface tensiometer (automatic contact angle meter DM-701, manufactured by Kyowa Interface Science Co., LTD.)

Viscosity

Viscosity at 25 degrees C of the support forming material in the present disclosure is preferably 1,000 mPa•s or less, more preferably 300 mPa•s or less, furthermore preferably 100 mPa•s or less, particularly preferably from 3 to 20 mPa•s, and most preferably from 6 to 12 mPa•s. With a viscosity surpassing 1,000 mPa•s, the support forming material may not be discharged even if a head is heated. Viscosity can be measured by, for example, a rotation viscometer (VISCOMATE VM-150 III, manufactured by TOKI SANGYO CO., LTD.) in a 25 degree C environment.

Removing Process and Removing Device

The removing process is to remove a support including the pillar-like core part.

The pillar-like core part can be removed due to heating causing liquefaction and using a solvent in which the tubular portion is insoluble. Being insoluble means that, for example, when 1 g of the tubular portion is mixed with 100 g of water at 30 degrees C and stirred, 90 percent by mass or more of the tubular portion is not dissolved in water.

Other Processes and Other Devices

There is no specific limitation to the other optional processes and it can be suitably selected to suit to a particular application.

Specific examples include, but are not limited to, a layer-smoothing process, a peeling-off process, discharging stabilizing process, a process of cleaning a fabricated object, and a process of polishing a fabricated object.

Method of Manufacturing Solid Freeform Fabrication Product and Device for Manufacturing Solid Freeform Fabrication Product

The method of manufacturing a solid freeform fabrication product of the present disclosure includes manufacturing a solid freeform fabrication product using the active energy ray curable liquid composition mentioned above and other optional processes.

In addition, the method of manufacturing a solid freeform fabrication product of the present disclosure includes laminating layers of the active energy ray curable liquid composition. The active energy ray curable liquid composition is laminated to form a cured object forming a support portion and the support portion is removed by heating after the additive manufacturing. Also, the method may include furthermore optional process.

Moreover, the device for manufacturing a solid freeform fabrication product in the present disclosure includes a container accommodating the active energy ray curable liquid composition, a discharging device to discharge the active energy ray curable liquid composition, a curing device to cure the active energy ray curable liquid composition discharged by the discharging device, and other optional devices.

As the active energy ray curable liquid composition, the same active energy ray curable liquid composition (support forming material for use in the additive manufacturing process in the method of manufacturing the hydrogel structure) mentioned above can be used.

In addition, as the method of manufacturing a solid freeform fabrication product, it is preferable that the cured object of the active energy ray curable liquid composition form a support portion and the hydrogel structure of the present disclosure form the model part in the additive manufacturing.

The container accommodating the active energy ray curable composition can be used as an ink cartridge and an ink bottle. This obviates the need for direct contact with ink in the operation of ink conveying, ink replacement, etc. so that contamination of fingers and clothes are prevented.

Furthermore, inclusion of foreign matters such as dust in the ink can be also prevented. In addition, the container has no particular limit. Size, form, and material of the container can be suitably selected to suit to a particular application and usage. For example, it is preferable to use a light blocking material to block the light or cover the container with a light blocking sheet, etc.

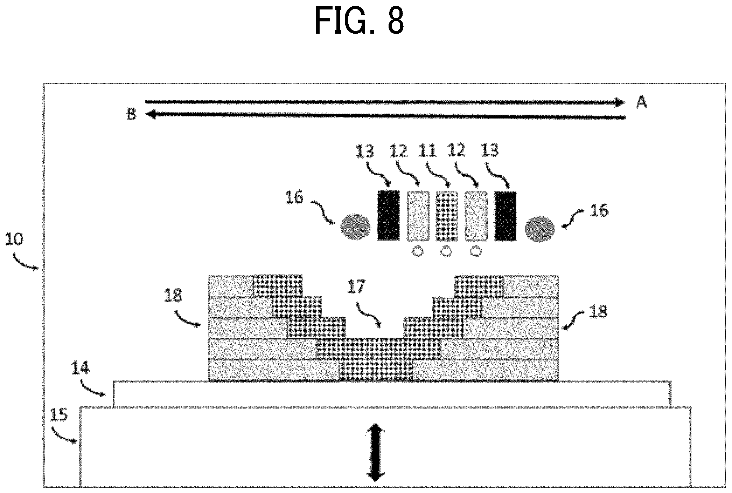

FIG. 8 is a schematic diagram illustrating an example of the process of manufacturing a solid freeform fabrication product using a device for manufacturing a solid freeform fabrication product for use in the method of manufacturing the solid freeform fabrication product of the present disclosure.

A solid freeform fabrication device 10 includes head units 11 and 12 in which inkjet heads (forming material discharging device, discharging device) movable in both directions indicated by the arrows A and B and a fabricated object supporting substrate 14. A hydrogel forming material is jetted from the head unit 12 and a support forming material is jetted from the head unit 11 on the fabricated object supporting substrate 14. The hydrogel forming material is laminated while cured by a UV radiation irradiator (curing device) 13 disposed adjacent to the head unit.

That is, the support forming material is jetted from the head unit 12 and solidified to form a first supporting layer having a pool part. Thereafter, the hydrogel forming material is jetted from the head unit 11 to the pool part of the first supporting layer and irradiated with UV radiation to cure the hydrogel forming material. Moreover, the cured part is smoothed by a smoothing member 16 to form a first solid freeform fabrication product layer.

Next, the support forming material is sprayed onto the first supporting layer and cured to stack a second supporting layer having a pool part. The hydrogel forming material is sprayed onto the pool part of the second supporting layer followed by irradiation of UV radiation to stack a second solid freeform fabrication product layer on the first solid freeform fabrication product layer followed by smoothing to manufacture a solid freeform fabrication product 17.

When the smoothing member having a roller form is used, it is preferable to reversely rotate the roller against the operation direction to ameliorate smoothing performance.

Furthermore, a stage 15 is lowered corresponding to the number of lamination to keep the gap constant between the head unit 11, the head unit 12, and the UV radiation irradiator 13 and the fabrication object 17 and a support 18.

In addition, the solid freeform fabrication device 10 may furthermore optionally include a mechanism for collecting and recycling the forming materials. Also, the solid freeform fabrication device 10 optionally includes a blade to remove the forming material attached to the nozzle surface and a detection mechanism to detect non-discharging nozzles. Moreover, it is preferable to control the environment temperature in the device during fabrication.

If the above-mentioned device is used, it is possible to control composition distribution and form control according to the state of a treatment site of a patient so that a blood vessel model or an internal organ model reflecting the form and property distribution peculiar to the patient.

Based on the personal data of a patient, it is possible to provide blood vessel forms of an affected part as a target of the catheter treatment and optionally a hardness distribution (composition distribution) of the blood vessels. Also in this case, the blood vessel is manufactured on the basis of the personal data of the patient.

One way of providing the composition distribution is to control the amount of the solvents contained in a hydrogel. This can be realized using a mechanism of discharging a plurality of compositions from respective inkjet heads utilizing the inkjet printing method described above.

A hydrogel forming material (hereinafter also referred to as liquid A) is discharged from a first head as a first liquid. A solvent (hereinafter also referred to as liquid B) capable of diluting the hydrogel forming material and mainly composed of water and a solvent soluble in water, is discharged from a second head as a second liquid. Moreover, a support forming material used to form a hollow tube in a blood vessel model is discharged as a third liquid from a third head.

The liquid A and the liquid B are discharged from each inkjet head in a predetermined amount of printing and the ratio of the liquids jetted onto the same site can be precisely controlled.

The method of manufacturing a solid freeform fabrication product of the present disclosure is described below with reference to specific embodiments.

The method of obtaining a hydrogel structure having different hardness, compression stress, and modulus of elasticity is described in detail.

First, surface data or solid data of a three-dimensional form designed by three dimensional computer-aided design (CAD) or taken in by a three-dimensional scanner or a digitizer are converted into Standard Template Library (STL) format, which is thereafter input into a additive manufacturing device.

Next, the compression stress distribution of the three dimensional form is measured. There is no specific limitation to methods of measuring the compression stress. For example, compression stress distribution data of a three dimensional form is obtained by using MR Elastography (MRE) and thereafter input into the additive manufacturing device. On the basis of the input compression stress data, the mixing ratio of the liquid A and the liquid B to be discharged to sites corresponding to the data of a three-dimensional form is determined.

On the basis of the input data, the direction of the fabrication of a three-dimensional form to be fabricated is determined.

The fabrication direction is not particularly limited. Normally, the direction is chosen such that the Z direction (height direction) is the lowest.

After determining the direction of fabrication, the projected areas on X-Y plane, X-Z plane, and Y-Z plane of the three-dimensional form are obtained. The thus-obtained block form is sliced in the Z direction with a thickness of a single layer. The thickness of a layer changes depending on the material and is normally from 20 to 60 $\mu$m. When only one three-dimensional product is manufactured, this block form is disposed in the center of the Z stage (i.e., table on which the product lifted down layer by layer for each layer forming is placed). In addition, when a plural of three-dimensional products are fabricated at the same time, the block forms are arranged on the Z stage. Alternatively, the block forms can be piled up. It is possible to automatically create these block forms, the slice data (contour line data), and the placement on the Z stage if materials to be used are determined.

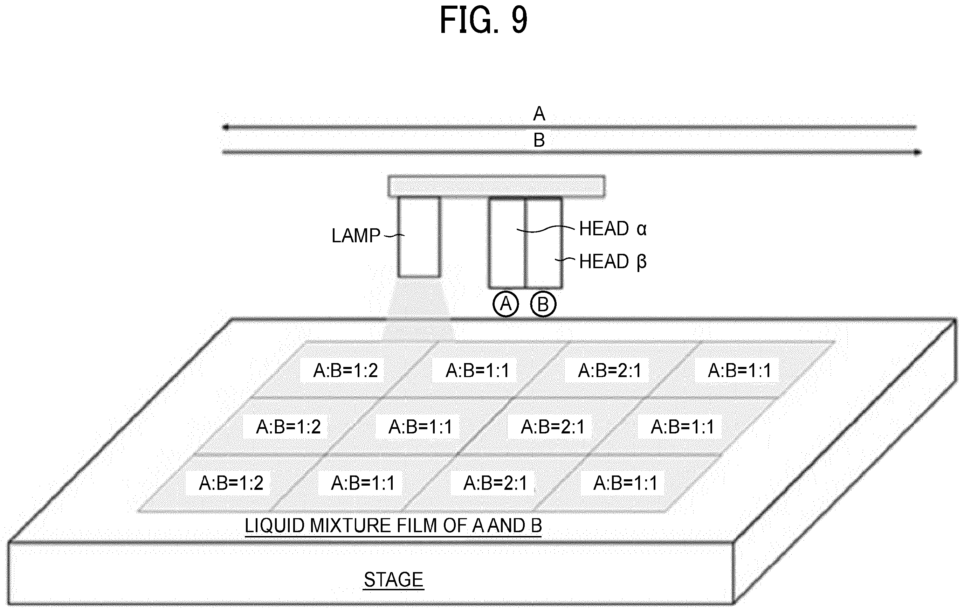

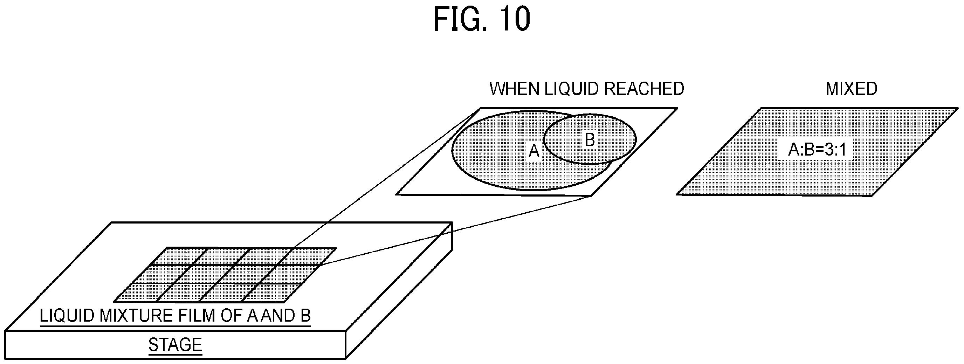

FIG. 9 is a schematic diagram illustrating an example in which the first liquid and the second liquid are mixed according to a liquid discharging method. Individual heads $\alpha$ and $\beta$ (illustrated in FIG. 8) are moved bi-directionally and discharge the liquid A and the liquid B to determined regions in a determined amount ratio to form a dot. The liquid A and the liquid B can be mixed in the dot as illustrated in FIG. 10 to obtain the pre-determined mixing ratio (liquid "A": liquid "B"). Moreover, such dots are continuously formed to form a liquid mixture film of the liquid A and the liquid B having the pre-determined mass ratio in the pre-determined area. Thereafter, the liquid mixture film is irradiated with ultraviolet (UV) radiation and cured to form a hydrogel film (layer) having the pre-determined ratio in the pre-determined region as illustrated in FIG. 9.

After a single layer of the hydrogel film (layer) is formed, the stage (FIG. 9) is lowered in an amount corresponding to the thickness of the single layer. Again, the dots are continuously formed on the hydrogel film to form a liquid mixture film of the liquid A and the liquid B having a pre-determined mass ratio in pre-determined regions. Thereafter, the liquid film of the liquid A and the liquid B is exposed to UV radiation and cured to form a hydrogel film. This lamination is repeated to form a three-dimensional object.

The thus-fabricated three-dimensional object (hydrogel object) has different mass ratios of liquid A and liquid B in the solid hydrogel of the liquid film illustrated in FIG. 8 so that modulus of elasticity therein can be continuously changed. If the mixing ratio pattern is adjusted for each cross section layer, a hydrogel structure partially having an arbitrary physical property can be obtained.

Furthermore, the UV radiation irradiator is disposed next to an inkjet head to jet a hydrogel forming material to save time to be taken for smoothing treatment, thereby speeding up the fabrication.

The hydrogel structure for use in the present disclosure can be arbitrarily changed in hardness using the same material if the composition ratio thereof is changed by the combination of the hydrogel forming material and diluting fluid. For this reason, in the case of fabrication according to an inkjet printing method, it is easy to provide the hardness distribution of a blood vessel based on personal data if the ratio of both is changed using a plurality of inkjet heads.

The hydrogel includes a massive amount of water and has a composition extremely close to a human body. Also, the texture thereof is very close as well. If this is used in combination with 3D printing, it is very useful to form a blood vessel model.

Since the hydrogel structure, the blood vessel model, and the internal organ model of the present disclosure can be manufactured by utilizing 3D printing technologies, a model reproducing the form and the property can be manufactured on the basis of the data of the diseased part of a patient. For this reason, it is suitable to use it for a simulation for delicate surgery.

For example, in typical operations (stent insertion into a swollen part), the form of the swollen part is read from an X-ray image and a stent considered having an appropriate form is selected and used during the operation. However, this relies on the experience of a doctor (surgeon). Therefore, in many cases, it took a long time before a decision or the most suitable stent was not selected.

If what form of a tool such as stent is to be selected is checked according to the form or the property of the swollen part before operation, the chance of success of the operation is expected to be high.

In the present disclosure, another embodiment of the blood vessel model or the internal organ model to which the technology of the present disclosure is applied is also disclosed.

The solid freeform fabrication product of the present disclosure has a feature of having a hollow part. To fabricate this structure, the hollow tubular structure is formed using the support forming material as described above. It is preferable to use a solid material that is phase-changed into liquid by heat as the support forming material.

When a blood vessel model and an internal organ model having the hollow structure are fabricated, fabrication is finished without removing the support with which the inside of the hollow structure is filled. As a result, while the hollow structure is maintained, the blood vessel model and the internal organ model in which the phase-changeable support forming material remains are manufactured. The support forming material used is preferably colored in red like blood by a colorant.

The blood vessel model and the internal organ model can be used in the training for surgical procedures with surgical tools such as ultrasonic wave knife and electrosurgical knife. More specifically, in the training of dissecting the site near the blood vessel disposed in an internal organ model, it can be used as an internal organ model from which blood bleeds if the blood vessel is mistakenly damaged.

Having generally described preferred embodiments of this disclosure, further understanding can be obtained by reference to certain specific examples which are provided herein for the purpose of illustration only and are not intended to be limiting. In the descriptions in the following examples, the numbers represent weight ratios in parts, unless otherwise specified.

EXAMPLES

Next, the present disclosure is described in detail with reference to Examples but is not limited thereto.

Example 1

Data Creation of Structure A and Structure B

Data for the hollow portion H is created on the basis of data of aortic valve and coronary artery. Aortic valve with coronary arteries 3D model (created by Surgen Pro and available from Turbosquad) was used as the data and selected in any range. The selected data is resized into 108 × 85 × 45 mm. The obtained data was divided into that for aortic valve portion V and coronary artery portion C1 with the hollow portion of the coronary artery portion C1 filled. This data was defined as the data of a coronary artery portion C2 (hollow portion H). Data was created to add a thickness of 2 mm to the coronary artery portion C2. The created data was defined as the data for a coronary artery portion C3. The data of the coronary artery portion C2 was booleaned from the data of the coronary artery portion C3 to create data for the tubular portion. This created data was defined as the data for a coronary artery portion C4. The structure A and the aortic valve portion V were smoothly connected (merged) while piercing the entrance of the hollow portion of the coronary artery portion C4 and the coronary artery of the aortic valve portion V.

"From top" of the structure A was determined. "From top" was the direction in which the aortic valve on the side of the heart was exposed. A pedestal was structured on the basis of this direction to obtain the data of the structure A and the data of the structure B. Refer to "Form of Structure B That Holds Structure A" described above for creation of the data of the Structure B.

Method of Manufacturing Mold (Core)

The form of the coronary artery portion C2 (hollow portion H) was output using a procured 3D printer (POLYGET, manufactured by Stratasys Ltd.) and molded using a silicone resin (KE-12, manufactured by Shin-Etsu Chemical Co., Ltd.). After the resin was completely cured, a sprue was provided to this mold. The mold was warmed in a hot bath at 80 degrees C for one hour. Molten wax (TOYO TECHNICAL WAX, HARD RED, manufactured by TOYO CHEMICAL LABORATORIES, INC.) was charged int the mold. The mold was placed in a thermostatic chamber at 120 degrees C and allowed to rest for eight hours followed by cooling down to cure the wax. The cured wax was taken out of the mold and the unwanted part such as burr of the wax was removed to obtain a core I made of wax with a form following the coronary artery portion C2.

Method of Manufacturing Mold (Exterior)

Molds of the structure A and the Structure were manufactured. The data of the structure A and the structure B were output by a 3D printer and the molds thereof were obtained using a silicone resin to obtain silicone resin molds. The obtained mold of the structure A and the obtained mold of the structure B were defined as mold Ma and Mold Mb, respectively.

Manufacturing of Structure A1 A releasing agent ("BARRIER COAT No. 7", manufactured by Shin-Etsu Chemical Co., Ltd.) was applied to the inside of the mold Ma and the core I made of wax was fixed onto a appropriate site of the mold Ma. A polyurethane precursor (HITOHADA® gel (transparent) hardness C7, manufactured by EXSEAL Co., Ltd.) was poured into the mold Ma from the sprue. The polyurethane precursor was sufficiently defoamed by stirring the main agent and a curing agent. After the precursor was sufficiently cured, the precursor was taken out of the mold Ma and placed in hot water at 65 degrees C to melt the core I made of wax. After the core I was removed, a liquid mixture of a coating agent (surface coating agent, manufactured by EXSEAL Co., Ltd.) and butyl acetate at a ratio of 1:1) was applied to the surface to obtain a structure A1 (moisture content: 0 percent, shore A hardness of 3).

Manufacturing of Structure B1

A releasing agent ("BARRIER COAT No. 7", manufactured by Shin-Etsu Chemical Co., Ltd.) was applied to the inside of the mold Mb. A polyurethane precursor (HITOHADA® gel (transparent) hardness C7) was poured into the mold Mb from the sprue. The polyurethane precursor was sufficiently defoamed by stirring the main agent and a curing agent. The polyurethane precursor was sufficiently cured at room temperature and taken out of the mold Mb. A liquid mixture of a coating agent (surface coating agent, manufactured by EXSEAL Co., Ltd.) and butyl acetate at a ratio of 1:1) was applied to the surface to obtain a structure B1 (moisture content: 0 percent, shore A hardness of 3).

Manufacturing of Set of Structure A1 and Structure B1

The structure A1 was inserted into and integrated with the structure B1 to obtain a set of the structure A1 and the structure B1.

Evaluation

Deformation and visibility of the structure A1 were evaluated regarding deformation by dead weight, deformation by external force, visibility from top, and visibility from oblique direction when a catheter (RINE CROSS MG, manufactured by Terumo Corporation) was inserted into the hollow portion H. The results are shown in Table 1.

Deformation by Dead Weight

The structure A1 or the set of the structure A1 and the structure B1 was placed on a glass plate (Blue (soda) glass, manufactured by AS ONE Corporation) and compared with the form on the data and evaluated the difference regarding the deformation by dead weight. The site where the difference was visible was measured with a ruler or caliper to analyze the degree of the error.

- • A: Deformation by dead weight was less than 2 mm
- • B: Deformation by dead weight was 2 mm or greater

Deformation by External Force

Whether the structure A1 was deformed under a force when a catheter was inserted into the structure A1.

- • A: Deformed
- • B: Not deformed

Visibility from Top

A catheter was inserted into the coronary artery of the structure A1. When the hollow portion H was viewed from top through the structure A1, how the gold marker attached to the catheter looked was checked.

- A: The contour of the gold marker was clearly seen
- B: The contour of the gold marker was blurred

Visibility from Oblique Direction

A catheter was inserted into the coronary artery of the structure A1. When the hollow portion H was viewed at 60 degrees shifted from top through the structure A1, how the gold marker attached to the catheter looked was checked.

- A: The contour of the gold marker was clearly seen
- B: The contour of the gold marker was blurred

Film Thickness

At least 10 portions were selected from the tubular portions of the fabrication product A and the film thickness at three points of each portion of from top and 60 degrees from top was measured. The average, the maximum, and the minimum of the measurements are shown in Table 1.

Comparative Example 1

The structure A1 alone was evaluated in the same manner as in Example 1. The evaluation results are shown in Table 1.

Comparative Example 2

The core I and the mold Mb were fixed with a cyanoacrylate-based glue (ARON ALPHA, manufactured by TOAGOSEI CO., LTD.) and a releasing agent was applied to the entire of both. The structure was placed in the polypropylene container (ZIPLOC, 480 ml, manufactured by Asahi Kasei Home Products Corporation) and 500 g of polyurethane precursor (HITOHADA® gel (transparent) hardness C7, manufactured by EXSEAL Co., Ltd.) in which the main agent and a curing agent were mixed was poured into the container to adjust the position of the core I.
At this point, the core I and the mold Mb were not in contact with the container. Also, the core I was not exposed to the liquid surface and part of the mold Mb was exposed to the liquid surface at this point.
While the core I was adjusted to keep the same position, the mold Mb and the core I were allowed to rest at room temperature for 48 hours. After the polyurethane was completely cured, the core I and the mold Mb were taken out from the container. A liquid mixture of a coating agent (surface coating agent, manufactured by EXSEAL Co., Ltd.) and butyl acetate at a mixing ratio of 1:1 was prepared and poured into the hollow portion H formed by taking out the core I followed by taking out and drying the liquid mixture. This procedure was repeated three times to obtain a structure A101 (moisture content: 0 percent, shore hardness A of 3 of cured product) having a hollow portion I. The structure A101 was evaluated in the same manner as in Example 1. The evaluation results are shown in Table 1.

Comparative Example 3

Hard material (VERO CLEAR, manufactured by Stratasys Ltd.) was output on the basis of the structure data A with a 3D printer available on the market to obtain a structure A102 (moisture content: 0 percent, shore hardness A of 100 of cured product). The structure A102 was evaluated in the same manner as in Example 1. The evaluation results are shown in Table 1.

Table 1

| | Form of structure | Material | Manufacturing method | Deformation by external force | Deformation by dead weight | Visibility from top |
|---|---|---|---|---|---|---|
| Example 1 | Set of tube and pedestal | Urethane | Silicone type cast molding | A | A | A |
| Comparative Example 1 | Tube | Urethane | Silicone type cast molding | A | B | A |
| Comparative Example 2 | Block | Urethane | Silicone type cast molding | A | A | A |
| Comparative Example 3 | Tube | Acrylic | Material jetting | B | A | A |

| | Visibility from oblique direction | Shore A hardness | Average film thickness (mm) | Minimum film thickness (mm) | Maximum film thickness (mm) |
|---|---|---|---|---|---|
| Example 1 | A | 3 | 2.13 | 2.05 | 2.26 |
| Comparative Example 1 | A | 3 | 2.15 | 2.06 | 2.24 |
| Comparative Example 2 | B | 3 | 2.16 | 2.08 | 2.28 |
| Comparative Example 3 | A | 100 | 1.99 | 1.97 | 2.03 |

The structure A of Comparative Example 1 readily deformed on its own because it did not have a structure B1. In particular, the front of the blood vessel deformed by dead weight so that the branch angle significantly changed.

In comparative Example 2, the core I and the mold Mb did not deform by dead weight because the hollow portion H was a block body.

However, the thickness to the hollow portion from top increased so that visibility deteriorated when the catheter was inserted into the hollow portion. This deterioration was significant when the structure A was viewed from the oblique direction.

The hard material of Comparative Example 3 did not deform on its own unlike comparative Example 1. However, it does not deform by an external force, so that the texture was significantly different from that of a live body when a catheter was inserted.

Example 2

Creation of Data for Integrated Structure A and Structure B

The coronary artery portion C2, coronary artery portion C3, aortic valve portion V, and the pedestal P1 and the cuboid R2 based on the structure A data were created in the same manner as in Example 1. Refer to "Form of Structure B That Holds Structure A" described above for creation of the pedestal P1 and the cuboid R2.

All of the coronary artery C3, the aortic valve portion V, the pedestal PI, and the cuboid R2 were merged and the coronary artery portion C2 was booleaned from the merged data. The outlet of the hollow portion H of the coronary artery portion and the inlet to the coronary artery of the aortic valve portion V were connected in such a manner that a catheter was able to be inserted to obtain a structure data AB as the integrated data of the structure A and the structure B.

Manufacturing of Structure AB1

Soft material (AGILUS 30, manufactured by Stratasys Ltd.) was output on the basis of the structure data AB with a 3D printer available on the market to obtain a structure AB1 (moisture content: 0 percent, shore hardness A of 32 of cured product). The structure AB1 was evaluated in the same manner as in Example 1. The evaluation results are shown in Table 2.

The structure AB1 shows good results about deformation by dead weight, deformation by external force, visibility from top, and visibility from oblique direction like Example 1. Moreover, there is no need for integrating the structure A and the structure B because the structure A and the structure B were integrally fabricated by material jetting. It is possible to readily obtain fabrication objects with a good accuracy because the structure AB1 was output with a 3D printer.

Example 3

The core I made of wax with a form of the coronary artery portion C2, the mold Ma as a set of the structure A mold, and the mold Mb as a set of the obtained structure B were manufactured in the same manner as in Example 1.

Manufacturing of Structure A2

A total of 15 g of agar powder (cool agar, produced by Nitta Gelatin Inc.) was slowly added dropwise at room temperature to 465 g of deionized water (also referred to as pure water) during stirring the deionized water.

After the addition, the system was heated to 95 degrees C during stirring. The temperature was held at 95 degrees C for five minutes. Thereafter, the resulting solution was rapidly cooled down with cooled water and subjected to deareation of air to obtain a hydrogel precursor 1.

The core I made of wax was fixed onto an appropriate site in the mold Ma and the hydrogel precursor 1 was poured from the sprue and allowed to rest at 4 degrees C for 24 hours. After the hydrogel precursor 1 was completely cured, it was taken out from the mold. The core I was extruded and removed to obtain a structure A2 (moisture content: 96.9 percent, shore A hardness of 0 of cured product).

Manufacturing of Structure B2

The hydrogel precursor 1 was poured from the sprue of the mold Mb and allowed to rest at 4 degrees C for 24 hours.

After the hydrogel precursor 1 was sufficiently cured, it was taken out from the mold to obtain a structure B2 (moisture content: 96.9 percent, shore A hardness of 0 of cured product).

Manufacturing of Set of Structure A2 and Structure B2

The structure A2 was inserted into and integrated with the structure B2 to obtain a set of the structure A2 and the structure B2. The set of the structure A2 and the structure B2 was evaluated in the same manner as in Example 1.

The evaluation results are shown in Table 2.

The set of the structure A2 and the structure B2 shows good results about deformation by dead weight, deformation by external force, visibility from top, and visibility from oblique direction like Example 1. The texture of the structure A was close to that of a live body when a catheter was inserted because the structure A was made of hydrogel.

Example 4

Preparation of Hydrogel Precursor 2

While stirring 120.0 parts of deionized water treated with degassing with a reduced pressure for 30 minutes, 12.0 parts of synthetic hectorite (LAPONITE XLG, manufactured by RockWood Additives Ltd.) having a composition of $[Mg_{5.34}Li_{0.66}Si_8O_{20}(OH)_4]Na^-_{0.66}$ as laminate clay mineral was added little by little to the deionized water followed by stirring. Moreover, 0.6 parts of etidronic acid (manufactured by Tokyo Chemical Industry Co. Ltd.) was added thereto to obtain a liquid dispersion.

Next, 44.0 parts of acryloyl morpholine (manufactured by KJ Chemicals Corporation) purged of the polymerization inhibitor by passage through an active alumina column and 0.4 parts of methylenebis acrylamide (manufactured by Tokyo Chemical Industry Co. Ltd.) were added as the polymerizable monomer to the thus-obtained liquid dispersion.

Moreover, 20.0 parts of glycerin (manufactured by Sakamoto Yakuhin Kogyo Co., Ltd.) and 0.8 parts of N,N,N',N'-tetramethylethylene diamine (manufactured by Tokyo Chemical Industry Co. Ltd.) were admixed to obtain a hydrogel precursor 2.

Method of Manufacturing Mold (Exterior)

A mold of the structure AB was manufactured. The form of the structure data AB was output by a 3D printer and the mold thereof was obtained using a silicone resin to obtain silicone resin mold Mab.

Manufacturing of Structure AB2

The core I made of wax was fixed onto an appropriate site in the mold Mab.

A total of 197.8 parts of the hydrogel precursor 2 was prepared and 1.2 parts of peroxo potassium pyrosulfate (manufactured by Wako Pure Chemical Industries, Ltd.) was poured thereto to melt the hydrogel precursor 2. The obtained liquid was poured into the sprue of the mold Mab and allowed to rest at room temperature for 24 hours under a sealed condition. After the liquid was completely cured, it was taken out from the mold. The core I was rinsed away with hexane heated to 60 degrees C to obtain a structure AB2 (moisture content: 60.6 percent, shore A hardness of 0 of cured product). The obtained structure AB2 was evaluated in the same manner as in Example 1. The evaluation results are shown in Table 2.

The structure AB2 shows good results about deformation by dead weight, deformation by external force, visibility from top, and visibility from oblique direction like Example 1. It is not necessary to combine the structure A and the structure B because both are integrated. The structure is not affected by melting the core with heat because the hydrogel constituting the structure A and the structure B does not change the phase by heat.

Example 5

Preparation of Hydrogel Precursor 3

While 120.0 parts of deionized water treated with degassing with a reduced pressure for 30 minutes was stirred, 12.0 parts of synthetic hectorite (LAPONITE XLG, manufactured by RockWood Additives Ltd.) having a composition of $[Mg_{5.34}Li_{0.66}Si_8O_{20}(OH)_4]Na^-_{0.66}$ as laminate clay mineral was slowly added to the deionized water followed by stirring. Moreover, 0.6 parts of etidronic acid (manufactured by Tokyo Chemical Industry Co. Ltd.) was added thereto to obtain a liquid dispersion.

Next, 44.0 parts of acryloyl morpholine (manufactured by KJ Chemicals Corporation) purged of the polymerization inhibitor by passage through an active alumina column and 0.4 parts of methylenebis acrylamide (manufactured by Tokyo Chemical Industry Co. Ltd.) were added as the polymerizable monomer to the thus-obtained liquid dispersion.

Moreover, 20.0 parts of glycerin (manufactured by Sakamoto Yakuhin Kogyo Co., Ltd.) and 0.8 parts of N,N,N',N'-tetramethylethylene diamine (manufactured by Tokyo Chemical Industry Co. Ltd.) were admixed.

A total of 1.2 parts of IRGACURE 184 (methanol solution at 4 percent by mass, manufactured by BASF SE) and 0.6 parts of SURFLON S-243 (manufactured by AGC SEIMI CHEMICAL CO., LTD.) to obtain a hydrogel precursor 3.

Manufacturing of Support Forming Material Precursor

A total of 58.0 parts of 1 dodecanol (Solvent (C), manufactured by Tokyo Chemical Industry Co. Ltd.), 20.0 parts of polypropylene glycol 2000 (Solvent (D), manufactured by Tokyo Chemical Industry Co. Ltd.), 28.0 parts of stearyl acrylate (polymer (A), manufactured by Tokyo Chemical Industry Co. Ltd.), and 4.0 parts of IRGACURE 819 (Polymerization initiator (B), manufactured by BASF) were stirred, mixed, and dissolved to prepare a precursor for support forming material.

Manufacturing of Structure AB3

The hydrogel precursor 3 and the precursor of support forming material were discharged using the fabrication device illustrated in FIG. 8 on the basis of the structure data AB and exposed to UV radiation to cure the materials. This process was repeated to obtain a laminated fabrication product. The laminated fabrication product was allowed to rest in a thermostatic chamber at 50 degrees C for 30 minutes to liquidize and remove the support. The pillar-like core part remaining was rinsed away with lukewarm water at 50 degrees C to obtain a structure AB3 (moisture content: 60.6 percent, shore A hardness of 0 of cured product). The obtained structure AB3 was evaluated in the same manner as in Example 1. The evaluation results are shown in Table 2.

The structure AB3 shows good results about deformation by dead weight, deformation by external force, visibility from top, and visibility from oblique direction like Example 1. Moreover, there is no need for integrating the structure A and the structure B because the structure A and the structure B are integrally fabricated by material jetting. It is possible to readily obtain fabrication objects with a good accuracy because the structure AB3 was output with a 3D printer. The structure is not affected by melting the support with heat because the hydrogel constituting the structure A and the structure B does not change the phase by heat.

Table 2

|  | Material | Structure | Manufacturing method | Material | Set | Hydrogel |
|---|---|---|---|---|---|---|
| Example 1 | Urethane | Structure A and structure B | Silicone type cast molding | Urethane | A | B |
| Example 2 | Photocurable rubber-like material | Integration of structure A and structure B | Material jetting | Photocurable rubber-like material | A | B |
| Example 3 | Agar | Structure A and structure B | Silicone type cast molding | Agar | A | A |

| | | | | | | |
|---|---|---|---|---|---|---|
| Example 4 | Nano composite gel | Integration of structure A and structure B | Silicone type cast molding | Nano composite gel | A | A |
| Example 5 | Nano composite gel | Integration of structure A and structure B | Material jetting | Nano composite gel | A | A |

| | Integrated | MJ | Shore A hardness | Average film thickness (mm) | Minimum film thickness (mm) | Maximum film thickness (mm) |
|---|---|---|---|---|---|---|
| Example 1 | B | B | 3 | 2.13 | 2.05 | 2.26 |
| Example 2 | A | A | 32 | 1.98 | 1.94 | 2.08 |
| Example 3 | B | B | 0 | 1.9 | 1.78 | 2.01 |
| Example 4 | A | B | 0 | 1.94 | 1.86 | 2.05 |
| Example 5 | A | A | 0 | 1.98 | 1.91 | 2.07 |

The present disclosure relates to the solid freeform fabrication product of the following 1 and also includes the following 2 to 7 as embodiments.

1. A solid freeform fabrication product includes a structure A containing a soft material and having a hollow portion H inside and a structure B retaining the structure A, wherein a layer between the structure A and the hollow portion H has a uniform thickness when he layer is viewed from top.

2. The solid freeform fabrication product according to 1 mentioned above, wherein the soft material contains a portion having a moisture content of 50 percent or more.

3. The solid freeform fabrication product according to 1 or 2 mentioned above, wherein the soft material contains a hydrogel contains a mineral, a polymer, and water.

4. The solid freeform fabrication product according to any one of 1 to 3 mentioned above, wherein the structure A and the structure B are integrally formed.

5. The solid freeform fabrication product according to 4 mentioned above, wherein, when the structure A is orthogonally projected onto a plane T in contact with the structure B, the figure formed on the plane T is defined as the figure S and when the figure created by intersections of perpendiculars from the plane T onto the structure A is defined as the figure S', the solid enclosed by the figure S and the figure S' is free of voids.

6. A method of manufacturing the solid freeform fabrication product of any one of 1 to 5 mentioned above includes discharging a first liquid precursor of a soft material from an inkjet head and exposing the first liquid precursor to ultraviolet radiation to cure the first liquid precursor.

7. The method according to 6 mentioned above further includes discharging a second liquid precursor of a support forming material from an inkjet head and exposing the second liquid precursor to ultraviolet radiation to cure the second liquid precursor.

Numerous additional modifications and variations are possible in light of the above teachings. It is therefore to be understood that, within the scope of the above teachings, the present disclosure may be practiced otherwise than as specifically described herein. With some embodiments having thus been described, it will be obvious that the same may be varied in many ways. Such variations are not to be regarded as a departure from the scope of the present disclosure and appended claims, and all such modifications are intended to be included within the scope of the present disclosure and appended claims.

**Claims**

1. A solid freeform fabrication product (100) comprising:

   a structure A (26) comprising a soft material and having a hollow portion H (21;24) inside; and
   a structure B (27) holding the structure A (26),
   wherein a layer between the structure A (26) and the hollow portion H (21;24) has a uniform thickness when the solid freeform fabrication product (100) is viewed from top.

2. The solid freeform fabrication product (100) according to claim 1, wherein the soft material comprises a portion having a moisture content of 50 percent or more.

3. The solid freeform fabrication product (100) according to claim 1 or 2, wherein the soft material comprises a hydrogel comprising a mineral, a polymer, and water.

4. The solid freeform fabrication product (100) according to any one of claim 1 to 3, wherein the structure A (26) and the structure B (27) are integrally formed.

5. The solid freeform fabrication product (100) according to 5, wherein, when a structure A (26) is orthogonally projected onto a plane T in contact with a structure B (27), a figure formed on the plane T is defined as a figure S and when a figure created by intersections of perpendiculars from the plane T onto the structure A (26) is defined as a figure S', a solid enclosed by the figure S and the figure S' is free of voids.

6. A method of manufacturing the solid freeform fabrication product of any one of claim 1 to 5, comprising: discharging a first liquid precursor of a soft material from an inkjet head; and exposing the first liquid precursor to ultraviolet radiation to cure the first liquid precursor.

7. The method according to claim 6, further comprising discharging a second liquid precursor of a support forming material from an inkjet head; and exposing the second liquid precursor to ultraviolet radiation to cure the second liquid precursor.

# FIG. 1

# FIG. 2

# FIG. 3

# FIG. 4A

# FIG. 4B

FIG. 5A

FIG. 5B

FIG. 6A

FIG. 6B

# FIG. 7A

# FIG. 7B

# FIG. 8

EP 3 827 966 A1

# FIG. 9

LABELS IN FIGURE:
- A
- B
- LAMP
- HEAD α
- HEAD β
- Ⓐ  Ⓑ
- A:B=1:2    A:B=1:1    A:B=2:1    A:B=1:1
- A:B=1:2    A:B=1:1    A:B=2:1    A:B=1:1
- A:B=1:2    A:B=1:1    A:B=2:1    A:B=1:1
- LIQUID MIXTURE FILM OF A AND B
- STAGE

EP 3 827 966 A1

# FIG. 10

WHEN LIQUID REACHED

MIXED

A    B

A:B=3:1

LIQUID MIXTURE FILM OF A AND B

STAGE

EP 3 827 966 A1

Europäisches
Patentamt
European
Patent Office
Office européen
des brevets

**EUROPEAN SEARCH REPORT**

Application Number

EP 20 21 0365

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | JP 2018 178069 A (RICOH CO LTD) 15 November 2018 (2018-11-15) * figures * * claims * * paragraph [0057] - paragraph [0065] * * paragraph [0057] - paragraph [0065] * * paragraph [0088] - paragraph [0222] * | 1-7 | INV. B29C64/112 B33Y80/00 |
| X | WO 2006/083963 A2 (SAKEZLES CHRISTOPHER [US]) 10 August 2006 (2006-08-10) * claims * * figures 1-6 * | 1-7 | |
| X | US 2013/102690 A1 (BATTERSBY RICHARD E [US] ET AL) 25 April 2013 (2013-04-25) * claims * * paragraph [0085] * | 1-7 | |
| X | WO 2015/144761 A1 (SQUARED GMBH I [CH]) 1 October 2015 (2015-10-01) * figure 1 * * page 26, line 20 - page 45, line 2 * | 1-4,6,7 | TECHNICAL FIELDS SEARCHED (IPC) B29C B33Y A61M G09B |
| X | CN 110 302 428 A (NANFANG HOSPITAL) 8 October 2019 (2019-10-08) * claims * * the whole document * | 1,2,4,6,7 | |
| X | CN 106 182 774 A (UNIV CENTRAL SOUTH) 7 December 2016 (2016-12-07) * figures * * claims * | 1,4,5 | |

The present search report has been drawn up for all claims

| Place of search | Date of completion of the search | Examiner |
|---|---|---|
| The Hague | 25 March 2021 | Whelan, Natalie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 21 0365

This annex lists the patent family members relating to the patent documents cited in the above-mentioned European search report.
The members are as contained in the European Patent Office EDP file on
The European Patent Office is in no way liable for these particulars which are merely given for the purpose of information.

25-03-2021

| Patent document cited in search report | | Publication date | Patent family member(s) | | Publication date |
|---|---|---|---|---|---|
| JP 2018178069 | A | 15-11-2018 | NONE | | |
| WO 2006083963 | A2 | 10-08-2006 | EP | 1848332 A2 | 31-10-2007 |
| | | | US | 2008187895 A1 | 07-08-2008 |
| | | | WO | 2006083963 A2 | 10-08-2006 |
| US 2013102690 | A1 | 25-04-2013 | AU | 2012325760 A1 | 24-04-2014 |
| | | | CA | 2852965 A1 | 25-04-2013 |
| | | | EP | 2758087 A1 | 30-07-2014 |
| | | | EP | 3417886 A1 | 26-12-2018 |
| | | | ES | 2687765 T3 | 29-10-2018 |
| | | | JP | 6162132 B2 | 12-07-2017 |
| | | | JP | 2014532458 A | 08-12-2014 |
| | | | PL | 2758087 T3 | 31-12-2018 |
| | | | US | 2013102690 A1 | 25-04-2013 |
| | | | US | 2015170547 A1 | 18-06-2015 |
| | | | WO | 2013059780 A1 | 25-04-2013 |
| WO 2015144761 | A1 | 01-10-2015 | EP | 3122537 A1 | 01-02-2017 |
| | | | JP | 6081401 B2 | 15-02-2017 |
| | | | JP | 2015183103 A | 22-10-2015 |
| | | | US | 2017001382 A1 | 05-01-2017 |
| | | | WO | 2015144761 A1 | 01-10-2015 |
| CN 110302428 | A | 08-10-2019 | NONE | | |
| CN 106182774 | A | 07-12-2016 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5140857 B
- JP 2009273508 A
- JP 6385664 B
- JP 2015069054 A
- JP 2018178069 A